# EUROPEAN PATENT APPLICATION

(11) **EP 2 384 772 A2**
(43) Date of publication of application: **09.11.2011**
(21) Application number: 11156493.6
(22) Date of filing: 21.11.2003
(51) Int. Cl.: A61L 12/08, G02B 1/04, G02C 7/04

(54) **Antimicrobial lenses, processes to prepare them and methods of their use**

(30) Priority: 22.11.2002 US 428620 P; 18.11.2003 US 715903
(62) Divisional of application: 03787028.4
(71) Applicant: Johnson & Johnson Vision Care, Inc., Jacksonville, FL 32256 (US)
(72) Inventor: Andersson, Ann Margaret, Hillsborough, NJ 08844 (US); Rathore, Osman, Basking Ridge, New Jersey 07920 (US); Meyers, Ann Marie Wong, Jacksonville, FL 32215 (US)
(74) Representative: Fisher, Adrian John

(57) **Abstract**

This invention relates to antimicrobial lenses containing metals and methods for their production.

## Description

### RELATED APPLICATIONS

This patent application claims priority of a provisional application, U.S. Ser. No. 60/428,620, which was filed on November 22, 2002.

### FIELD OF THE INVENTION

This invention relates to antimicrobial lenses as well as methods of their production, and use.

### BACKGROUND OF THE INVENTION

Contact lenses have been used commercially to improve vision since the 1950s. The first contact lenses were made of hard materials. They were used by a patient during waking hours and removed for cleaning. Current developments in the field gave rise to soft contact lenses, which may be worn continuously, for several days or more without removal for cleaning. Although many patients favor these lenses due to their increased comfort, these lenses can cause some adverse reactions to the user. The extended use of the lenses can encourage the buildup of bacteria or other microbes, particularly, *Pseudomonas aeruginosa*, on the surfaces of soft contact lenses. The build-up of bacteria and other microbes can cause adverse side effects such as contact lens acute red eye and the like. Although the problem of bacteria and other microbes is most often associated with the extended use of soft contact lenses, the build-up of bacteria and other microbes occurs for users of hard contact lens wearers as well.

US 5,820,918 discloses medical devices made from a water absorbable polymer material with a medical compound having low solubility in aqueous solutions such as an antiseptic or radiopaque compound. However, the procedures disclosed in the examples yield opaque devices which are not suitable for ophthalmic devices such as contact lenses.

Therefore, there is a need to produce contact lenses that inhibit the growth of bacteria or other microbes and/or the adhesion of bacteria or other microbes on the surface of contact lenses. Further there is a need to produce contact lenses which do not promote the adhesion and/or growth of bacteria or other microbes on the surface of the contact lenses. Also there is a need to produce contact lenses that inhibit adverse responses related to the growth of bacteria or other microbes.

### BRIEF DESCRIPTION OF THE DRAWINGS

- Figure 1: Plot of silver release over thirty days
- Figure 2: Plot of silver release over thirty days
- Figure 3: Plot of silver release over thirty days
- Figure 4: Plot of silver release over thirty days
- Figure 5: Plot of silver release over thirty days
- Figure 6: Plot of silver release over thirty days
- Figure 7: Plot of silver release over thirty days
- Figure 8: Plot of silver concentration vs. autoclave cycle
- Figure 9: Plot of silver concentration vs. autoclave cycle
- Figure 10: Plot of silver release over thirty days
- Figure 11: Plot of silver release over thirty days
- Figure 12: Plot of silver release over thirty days
- Figure 13: Plot of silver release over thirty days
- Figure 14: Plot of silver concentration vs. silverizing time
- Figure 15: Plot of silver release over thirty days
- Figure 16: Plot of silver release over thirty days
- Figure 17: Plot of silver release over twenty-one days
- Figure 18: Plot of silver release over twenty-one days
- Figure 19: Plot of silver release over thirty days
- Figure 20: Plot of silver release over thirty days
- Figure 21: Plot of silver release over thirty days

### DETAILED DESCRIPTION OF THE INVENTION

This invention includes an antimicrobial lens comprising, consisting essentially of, or consisting of a metal salt. As used herein, the term, "antimicrobial lens" means a lens that exhibits one or more of the following properties, the inhibition of the adhesion of bacteria or other microbes to the lenses, the inhibition of the growth of bacteria or other microbes on lenses, and the killing of bacteria or other microbes on the surface of lenses or in an area surrounding the lenses. For purposes of this invention, adhesion of bacteria or other microbes to lenses, the growth of bacteria or other microbes on lenses and the presence of bacteria or other microbes on the surface of lenses are collectively referred to as "microbial colonization." Preferably, the lenses of the invention exhibit a reduction of viable bacteria or other microbe of at least about 0.25 log, more preferably at least about 0.5 log, most preferably at least about 1.0 log (≥ 90% inhibition). Such bacteria or other microbes include but are not limited to those organisms found in the eye, particularly *Pseudomonas aeruginosa, Acanthamoeba species, Staphyloccus*. *aureus*, *E. coli, Staphyloccus epidermidis*, and *Serratia marcesens*.

As use herein, the term "metal salt" means any molecule having the general formula [M]ₐ [X]_{b} wherein X contains any negatively charged ion, a is ≥ 1, b is ≥ 1 and M is any positively charged metal selected from, but not limited to, the following Al⁺³, Co⁺², Co⁺³, Ca⁺², Mg⁺² , Ni⁺² , Ti⁺², Ti⁺³, Ti⁺⁴, V⁺², V⁺³, V⁺⁵, Sr⁺², Fe⁺², Fe⁺³, Au⁺², Au⁺³, Au⁺¹, Pd⁺², Pd⁺⁴, Pt⁺², Pt⁺⁴, Cu⁺¹, Cu⁺², Mn⁺², Mn⁺³, Mn⁺⁴, Zn⁺², and the like. Examples of X include but are not limited to CO₃⁻², NO₃⁻¹, PO₄⁻³ , Cl⁻¹, I⁻¹, Br⁻¹, S⁻², O⁻² and the like. Further X includes negatively charged ions containing CO₃⁻² NO₃⁻¹, PO₄⁻³, Cl⁻¹, I⁻¹, Br⁻¹, S⁻², O⁻², and the like, such as C₁₋₅alkylCO2⁻¹. As used herein the term metal salts does not include zeolites, disclosed in WO03/011351. This patent application is hereby incorporated by reference in its entirety. The preferred a is 1, 2, or 3. The preferred b is 1, 2, or 3. The preferred metals ions are Mg⁺², Zn⁺², Cu⁺¹, Cu⁺² , Au⁺², Au+³, Au⁺¹, Pd⁺², Pd⁺⁴, Pt⁺², Pt⁺⁴, Ag⁺², and Ag⁺¹. The particularly preferred metal ion is Ag⁺¹. Examples of suitable metal salts include but are not limited to manganese sulfide, zinc oxide, zinc sulfide, copper sulfide, and copper phosphate. Examples of silver salts include but are not limited to silver nitrate, silver sulfate, silver iodate, silver carbonate, silver phosphate, silver sulfide, silver chloride, silver bromide, silver iodide, and silver oxide. The preferred silver salts are silver iodide, silver chloride, and silver bromide. The lenses of the invention are ophthalmic lenses (a detailed description of these lenses follows) and the clarity of the lenses is of concern to users. In order to produce lenses having a clarity suitable for ophthalmic purposes, it is preferred that the diameter of the metal salt particles is less than about ten microns (10 µm), more preferably less than about 5 µm, most preferably equal to or less than about 200 nm.

The amount of metal in the lenses is measured based upon the total weight of the lenses. When the metal is silver, the preferred amount of silver is about 0.00001 weight percent (0.1 ppm) to about 10.0 weight percent, preferably about 0.0001 weight percent (1 ppm) to about 1.0 weight percent, most preferably about 0.001 weight percent (10 ppm) to about 0.1 weight percent, based on the dry weight of the lens. With respect to adding metal salts, the molecular weight of the metal salts determines the conversion of weight percent of metal ion to metal salt. The preferred amount of silver salt is about 0.00003 weight percent (0.3 ppm) to about 30.0 weight percent, preferably about 0.0003 weight percent (3 ppm) to about 3.0 weight percent, most preferably about 0.003 weight percent (30 ppm) to about 0.3 weight percent, based on the dry weight of the lens.

As used herein, the term "lens" refers to an ophthalmic device that resides in or on the eye. These devices can provide optical correction, wound care, drug delivery, diagnostic functionality, cosmetic enhancement or effect or a combination of these properties. The term lens includes but is not limited to soft contact lenses, hard contact lenses, intraocular lenses, overlay lenses, ocular inserts, and optical inserts. Soft contact lenses are made from silicone elastomers or hydrogels, which include but are not limited to silicone hydrogels, and fluorohydrogels. Preferably, the lenses of the invention are optically clear, with optical clarity comparable to lenses such as lenses made from etafilcon A, genfilcon A, lenefilcon A, polymacon, acquafilcon A, balafilcon A, and lotrafilcon A.

It has been found that when the metal salt is incorporated in accordance with the teachings of the present invention, ophthalmic devices that are substantially free from unwanted haze are produced. Specifically, lenses of the present invention have a percent haze that is less than about 200%, preferably less than about 150% and more preferably less than about 100%. Percent haze is measured using the following method. The haze is measured by placing a hydrated test lens in borate buffered saline in a clear 20 x 40 x 10 mm glass cell at ambient temperature above a flat black background illuminating from below with a fiber optic lamp (Titan Tool Supply Co. fiber optic light with 0.5" diameter light guide set at a power setting of 4-5.4) at an angle 66° normal to the lens cell, and capturing an image of the lens from above from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The -subtracted scattered light image is quantitatively analyzed, by integrating over the central 10 mm of the lens, and then comparing to a CSI Thin Lens®, (CSI Flexible Wear (crotofilcon A) lot ML 62900207 Power-1.0) which is arbitrarily set at a haze value of 100. Four lenses are analyzed and the results are averaged to generate a haze value as a percentage of the standard CSI lens.

Metal salts of the invention may be added (prior to curing) to the soft contact lens formulations described in US 5,710,302, WO 9421698, EP 406161, JP 2000016905, U.S. 5,998,498, US Pat. App. No. 09/532,943, U.S. 6,087,415, U.S. 5,760,100, U.S.5,776,999, U.S. 5,789,461, U.S. 5,849,811, and U.S. 5,965,631. In addition, metal salts of the invention may be added to the formulations of commercial soft contact lenses. Examples of soft contact lenses formulations include but are not limited to the formulations of etafilcon A, genfilcon A, lenefilcon A, polymacon, acquafilcon A, balafilcon A, galyfilcon A, senofilcon A and lotrafilcon A. The preferable contact lens formulations are etafilcon A, balafilcon A, acquafilcon A, lotrafilcon A, and silicone hydrogels, as prepared in U.S. 5,998,498, US Ser. No. 09/532,943, a continuation-in-part of US Pat App. No. 09/532,943, filed on August 30, 2000, WO03/22321, U.S. 6,087,415, U.S. 5,760,100, U.S. 5,776, 999, U.S. 5,789,461, U.S. 5,849,811, and U.S. 5,965,631. These patents as well as all other patent disclosed in this paragraph are hereby incorporated by reference in their entirety.

Hard contact lenses are made from polymers that include but are not limited to polymers of poly(methyl)methacrylate, silicon acrylates, silicone acrylates, fluoroacrylates, fluoroethers, polyacetylenes, and polyimides, where the preparation of representative examples may be found in JP 200010055, JP 6123860 and U.S. Patent 4,330,383. Intraocular lenses of the invention can be formed using known materials. For example, the lenses may be made from a rigid material including, without limitation, polymethyl methacrylate, polystyrene, polycarbonate, or the like, and combinations thereof. Additionally, flexible materials may be used including, without limitation, hydrogels, silicone materials, acrylic materials, fluorocarbon materials and the like, or combinations thereof. Typical intraocular lenses are described in WO 0026698, WO 0022460, WO 9929750, WO 9927978, WO 0022459, and JP 2000107277. U.S. 4,301,012; 4,872,876; 4,863,464; 4,725,277; 4,731,079. Metal salts may be added to hard contact lens formulations and intraocular lens formulations in the same manner (prior to curing) as soft contact lenses. All of the references mentioned in this application are hereby incorporated by reference in their entirety.

Preferably the silver releasing compounds are added to silicone hydrogel formulations or contact lenses made therefrom. A silicone-containing component is one that contains at least one [-Si-O-Si] group, in a monomer, macromer or prepolymer. Preferably, the Si and attached O are present in the silicone-containing component in an amount greater than 20 weight percent, and more preferably greater than 30 weight percent of the total molecular weight of the silicone-containing component. Useful silicone-containing components preferably comprise polymerizable functional groups such as acrylate, methacrylate, acrylamide, methacrylamide, N-vinyl lactam, N-vinylamide, and styryl functional groups. Examples of silicone components which may be included in the silicone hydrogel formulations include, but are not limited to silicone macromers, prepolymers and monomers. Examples of silicone macromers include, without limitation, polydimethylsiloxane methacrylated with pendant hydrophilic groups as described in United States Patents Nos. 4,259,467; 4,260,725 and 4,261,875; polydimethylsiloxane macromers with polymerizable functional group(s) described in U.S. Patents Nos. 4,136,250; 4,153,641; 4,189,546; 4,182,822; 4,343,927; 4,254,248; 4,355,147; 4,276,402; 4,327,203; 4,341,889; 4,486,577; 4,605,712; 4,543,398; 4,661,575; 4,703,097; 4,837,289; 4,954,586; 4,954,587; 5,346,946; 5,358,995; 5,387,632 ; 5,451,617; 5,486,579; 5,962,548; 5,981,615; 5,981,675; and 6,039,913; polysiloxane macromers incorporating hydrophilic monomers such as those described in U.S. Patents Nos. 5,010,141; 5,057,578; 5,314,960; 5,371,147 and 5,336,797; macromers comprising polydimethylsiloxane blocks and polyether blocks such as those described in U.S. Patents Nos. 4,871,785 and 5,034,461, combinations thereof and the like. All of the patents cited herein are hereby incorporated in their entireties by reference.

The silicone and/or fluorine containing macromers described in U.S. Patents Nos. 5,760,100; 5,776,999; 5,789,461; 5,807,944; 5,965,631 and 5,958,440 may also be used. Suitable silicone monomers include tris(trimethylsiloxy)silylpropyl methacrylate, hydroxyl functional silicone containing monomers, such as 3-methacryloxy-2-hydroxypropyloxy)propylbis(trimethylsiloxy)methylsilane and those disclosed in WO03/22321, and mPDMS containing or the siloxane monomers described in U.S. Patents Nos. 4,120,570, 4,139,692, 4,463,149, 4,450,264, 4,525,563; 5,998,498; 3,808,178; 4,139,513; 5,070,215; 5,710,302; 5,714,557 and 5,908,906.

Additional suitable siloxane containing monomers include, amide analogs of TRIS described in U.S. 4,711,943, vinylcarbamate or carbonate analogs decribed in U.S. 5,070,215, and monomers contained in U.S. 6,020,445, monomethacryloxypropyl terminated polydimethylsiloxanes, polydimethylsiloxanes, 3-methacryloxypropylbis(trimethylsiloxy)methylsilane, methacryloxypropylpentamethyl disiloxane and combinations thereof.

Often lenses are coated to increase their compatibility with living tissue. Therefore, the lenses of the inventions may be coated with a number of agents that are used to coat lens. For example, the coating procedures, compositions, and methods of WO03/11551, U.S. 6,087,415, 5,779,943, 5,275,838, 4,973,493, 5,135,297, 6,193,369, 6,213,604, 6,200,626, and 5,760,100 may be used and these applications and patents are hereby incorporated by reference for those procedures, compositions, and methods.

Many of the lens formulations cited above may allow a user to insert the lenses for a continuous period of time ranging from one day to thirty days. It is known that the longer a lens is on the eye, the greater the chance that bacteria and other microbes will build up on the surface of those lenses. Therefore there is a need to develop lenses that release antimicrobial agents such as silver, over an extended period of time.

The invention includes an antimicrobial lens comprising, consisting of, or consisting essentially of, a metal salt wherein the molar solubility of the metal ion in pure water at about 25°C is greater than about 2.0 x 10⁻³⁰ moles/L to about less than about 20 moles/L. The preferred metal salts are silver salts where the silver ion has a molar solubility of greater than about 2.0 x 10⁻¹⁷ moles/L.

The terms antimicrobial lens and metal salt have their aforementioned meanings and preferred ranges. As used herein, the term "pure" refers to the quality of the water used as defined in the CRC Handbook of Chemistry and Physics, 74th Edition, CRC Press, Boca Raton Florida, 1993. The term "molar solubility" refers to the number of moles of metal dissolved or dissociated from the anion per liter of water. This number is derived from the solubility-product constant (Kₛₚ) measured in pure water at 25°C. (See Skoog, D.A. et al. FUNDAMENTALS OF ANALYTICAL CHEMISTRY, Fifth Edition, Saunders College Publishing, New York, 1988, see also, published values in CRC Handbook of Chemistry and Physics, 74th Edition, CRC Press, Boca Raton Florida, 1993) For example, if the metal salt is silver carbonate (Ag₂CO₃) the Kₛₚ is expressed by the following equation

Ag₂CO₃(s) → 2Ag⁺(*aq*) + CO₃²⁻(*aq*)

The Kₛₚ is calculated as follows

Kₛₚ = [Ag⁺]² [CO₃²]

As silver carbonate dissolves, there is one carbonate anion in solution for every two silver cations, [CO₃²⁻] = ½[Ag⁺], and the solubility-product constant equation can be rearranged to solve for the dissolved silver concentration as follows

*K*ₛₚ = [Ag⁺]²(1/2[Ag⁺]) =½[Ag⁺]³

[Ag⁺] = (2Kₛₚ)^{1/3}

The Kₛₚ may be used to calculate the molar solubility of any metal salt as follows

For MX: [M] = (*K*ₛₚ)^{1/2}

For M₂X: [M] = (2*K*ₛₚ)^{1/3}

For M₃X: [M] = (3*K*ₛₚ)^{1/4}

It has been discovered that certain metal salts wherein the metal ion has a molar solubility of about greater than about 2 x 10⁻³⁰ moles/L to less than about 20 moles/L when measured at 25°C will continuously release the metal from lenses for a period of time from one day to up to or longer than a thirty day period. The preferred metal salts of the invention are silver salts, wherein the molar solubility of the silver ion is greater than or equal to about 2 x 10⁻¹⁷ moles/L. The preferred molar solubility is greater than or equal to about 9 x 10⁻⁹ moles/L to less than or equal to 1 x 10⁻⁵ moles/L when measured at 25°C. The preferred metal salts of this invention are silver iodide, silver chloride and silver bromide, where silver iodide is particularly preferred.

Still further, the invention includes an antimicrobial lens comprising, consisting of, or consisting essentially of, a metal complex wherein the molar solubility of the metal ions in water at about 25°C is greater than about 2 x 10⁻³⁰ moles/L. The terms antimicrobial lens and molar solubility have their aforementioned meanings and preferred ranges. The term "metal complex" means any molecule or compound that is not covalently incorporated into the lens matrix and contains a positively charged metal ion, wherein the metal ions are selected from but not limited to any of the following aluminum, cobalt, calcium, magnesium, manganese, zinc, nickel, titanium, vanadium, strontium, iron, gold, silver, palladium, and platinum, where silver is the preferred metal ion. The term metal complex does not include zeolites as described in WO03/11351; the silver ceramics as disclosed in U.S. Pat. No. 6,143,318, U.S. 5,470,585; WO 90/08470, U.S. 5,213,801, WO 89/01793. All of the aforementioned patent application are hereby incorporated by reference in their entirety.

Still yet further, the invention includes a method of reducing the adverse events associated with microbial colonization on a lens placed in the ocular regions of a mammal comprising, consisting of, or consisting essentially of, placing an antimicrobial lens comprising a metal salt on the eye of a mammal. The terms lens, antimicrobial lens, and metal salt all have their aforementioned meanings and preferred ranges. The phrase "adverse events associated with microbial colonization" include but are not limited to contact ocular inflammation, contact lens related peripheral ulcers, contact lens associated red eye, infiltrative keratitis, microbial keratitis, and the like. The term mammal means any warm blooded higher vertebrate, and the preferred mammal is a human.

Still yet even further, the invention includes a method of producing an antimicrobial lens comprising, consisting essentially of, or consisting of a metal salt wherein the method comprises, consists essentially of or consists of mixing the metal salt with a lens formulation and curing the lens formulation/metal salt mixture to form a lens. The terms antimicrobial lens and metal salt have their aforementioned meanings and preferred ranges. The term "formulation" includes any ingredient or combination of ingredients that is used to make antimicrobial lenses, such as monomer, pre-polymers, co-polymers, macromers, initiators, pigments, dyes, UV absorbing agents and the like. Examples of such ingredients are known in the art and some of those ingredients are disclosed in the ophthalmic lens patents and patent applications cited earlier in this application.

Even further, the invention includes a method of preparing an antimicrobial lens comprising, consisting essentially of, or consisting of a metal salt, wherein the method comprises, consists essentially of, or consists of the steps of
(a) mixing a salt precursor with a lens formulation;
(b) forming the lens; and
(c) treating the lens with a metal agent.

The terms antimicrobial lens, metal salt, and lens formulation all have their aforementioned meanings and preferred ranges. The term "salt precursor" refers to any compound or composition (including aqueous solutions) that contains a cation that may be substituted with metal ions. It is preferred that the salt precursor is soluble in lens formulation at about 1 µg/mL or greater. The term salt precursor does not include zeolites as described in WO03/11351, solid silver as described in WO02/62402. The preferred amounts of salt precursor in the lens is about 0.00001 to about 10.0 weight percent, more preferably about 0.0001 to about 1.0 weight percent, most preferably about 0.001 to about 0.1 weight percent based upon the total weight of the monomer composition. Examples of salt precursors include but are not limited to inorganic molecules such as sodium chloride, sodium iodide, sodium bromide, lithium chloride, lithium sulfide, sodium sulfide, potassium sulfide, sodium tetrachloro argentate, and the like. Examples of organic molecules include but are not limited to tetra-alkyl ammonium lactate, tetra-alkyl ammonium sulfate, quaternary ammonium halides, such as tetra-alkyl ammonium chloride, bromide or iodide. The preferred precursor salt is sodium iodide.

The term "forming" refers to any of a number of methods used to form lenses that include but are not limited to curing with light or heat. The lens formulations of the present invention can be formed by any of the methods know to those skilled in the art, such as shaking or stirring, and used to form polymeric articles or devices by known methods.

For example, the ophthalmic devices of the invention may be prepared by mixing reactive components and any diluent(s) with a polymerization initator and curing by appropriate conditions to form a product that can be subsequently formed into the appropriate shape by lathing, cutting and the like. Alternatively, the reaction mixture may be placed in a mold and subsequently cured into the appropriate article.

Various processes are known for processing the lens formulation in the production of contact lenses, including spincasting and static casting. Spincasting methods are disclosed in U.S. 3,408,429 and 3,660,545, and static casting methods are disclosed in U.S. 4,113,224 and 4,197,266. The preferred method for producing contact lenses of this invention is by molding. For this method, the lens formulation is placed in a mold having the shape of the final desired lens, and the lens formulation is subjected to conditions whereby the components polymerize, to produce a lens. The lens may be treated with a solvent to remove the diluent and ultimately replace it with water. This method is further described in U.S. Pat. Nos. 4,495,313; 4,680,336; 4,889,664; and 5,039,459, incorporated herein by reference. The preferred method of curing is with radiation, preferably UV or visible light, and most preferably with visible light.

The term "metal agent" refers to any composition (including aqueous solutions) containing metal ions. Examples of such compositions include but are not limited to aqueous or organic solutions of silver nitrate, silver triflate, or silver acetate, where the concentration of metal agent in solution is about 1µg/mL or greater. The preferred metal agent is aqueous silver nitrate, where the concentration of silver nitrate is the solution is about greater than or equal to 0.0001 to about 2 weight percent, more preferably about greater than 0.001 to about 0.01 weight percent based on the total weight of the solution. The term "treating" refers to any method of contacting the metal agent with the lens, where the preferred method is immersing the lens in a solution of the metal agent. Treating can include heating the lens in a solution of the metal agent, but it preferred that treating is carried out at ambient temperatures.

Yet even further, the invention includes a method of preparing an antimicrobial lens comprising, consisting essentially of, or consisting of a metal salt, wherein the method comprises, consists essentially of, or consists of the steps of
(a) mixing a metal precursor with a lens formulation;
(b) forming the lens; and
(c) treating the lens with an anion precursor.

The terms antimicrobial lens, forming, and treating all have their aforementioned meanings and preferred ranges. The term metal precursor refers to any composition (including aqueous solutions) that contains a metal cation and a counter anion wherein the counter anion may be substituted. Examples of metal precursors include but are not limited to silver triflate, copper nitrate, copper sulfate, magnesium sulfate, zinc sulfate, and the like. The preferred metal precursor is silver triflate. The term anion precursor refers to any composition (including aqueous solutions) that contains an anion that may be substituted with the anion of the metal precursor to form a metal salt. Examples of anion precursors include but are not limited to inorganic molecules such as sodium chloride, sodium iodide, sodium bromide, lithium chloride, lithium sulfide, sodium sulfide, potassium sulfide and the like. Examples of anion precursors that are organic molecules include but are not limited to tetra-alkyl ammonium lactate, tetra-alkyl ammonium sulfate, quaternary ammonium halides, such as tetra-alkyl ammonium chloride, bromide or iodide. The preferred anion precursor is aqueous sodium iodide.

And yet even further, the invention includes a method of preparing an antimicrobial lens comprising, consisting essentially of, or consisting of a metal salt, wherein the method comprises, consists essentially of, or consists of the steps of
(a) treating a cured lens with a salt precursor;
(b) treating the lens of step (a) with a metal agent.
The terms antimicrobial lens, salt precursor, metal agent, and treating all have their aforementioned meanings and preferred ranges.

Still, yet even further, the invention includes a method of preparing an antimicrobial lens comprising, consisting essentially of, or consisting of a metal salt, wherein the method comprises, consists essentially of, or consists of the steps of
(a) mixing a metal with a lens formulation;
(b) forming the lens;
(c) treating the lens of step (b) with an oxidizing agent; and
(d) treating the lens of step (c) with an anion precursor.

The terms antimicrobial lens, anion precursor, forming, and treating, all have their aforementioned meanings and preferred ranges. The term "metal" refers to any metal having an oxidation state of zero. Examples of metals include but are not limited to aluminum, cobalt, calcium, magnesium, nickel, titanium, vanadium, strontium, iron, gold, silver, palladium, platinum, copper, manganese and zinc. The preferred metals are manganese, zinc, copper, gold, platinum, palladium and silver, the particularly preferred metal is silver. The term "oxidizing agent" includes but in not limited know agents such as hydrogen peroxide and the like.

All of the aforementioned processes may be carried out by a single mechanical device or a combination of mechanical devices. For example, if metal salts are added to cured lenses, all of the steps to add those metal salts may be carried out on a hydration machine which functions as follows. A cured lens (non-hydrated, partially hydrated or fully hydrated lens) may be placed in a single blister package. A solution of a salt precursor is added to this package and left for a time sufficient to allow the desired amount of salt precursor to be incorporated into the lens, but insufficient to produce discoloration or haze. The time will vary depending upon the solubility and concentration of the salt and temperature. Suitable times (at ambient temperature) include,up to about 30 minutes and preferably between about 30 seconds and 5 minutes and more preferably about two minutes. Subsequently, the solution of the salt precursor is removed and a solution of a metal agent is added to the package. The soak time for the metal agent can be selected using the solubility, concentration and temperature. Subsequently the metal agent solution is removed and the lens is washed with several portions deionized water, followed by sterilization.

Still yet even further, the invention includes a method of preparing an antimicrobial lens comprising, consisting essentially of, or consisting of a metal salt, wherein the method comprises, consists essentially of, or consists of the steps of
(a) treating a cured lens with a metal agent.
(b) treating the lens of step (a) with a salt precursor;

The terms antimicrobial lens, salt precursor, metal agent, and treating all have their aforementioned meanings and preferred ranges.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

### EXAMPLES

The following abbreviations were used in the examples
Bloc-HEMA = 2-(trimethylsiloxy) ethyl methacrylate
Blue HEMA = the reaction product of reactive blue number 4 and HEMA, as described in Example 4 or U.S. Pat. no. 5,944,853
CGI 1850 = 1:1 (w/w) blend of 1-hydroxycyclohexyl phenyl ketone and bis (2,6-dimethyoxybenzoyl)-2,4-4-trimethylpentyl phosphine oxide
DI water = deionized water
D30 = 3,7-dimethyl-3-octanol
DMA = N,N-dimethylacrylamide
DAROCUR 1173 2-hydroxy-2-methyl-1-phenyl-propan-1-one
DPMA = di(propyleneglycol)methyl ether acetate
DPM = dipropylene glycol monomethyl ether
EGDMA = ethyleneglycol dimethacrylate
EO₂V = diethylene glycol vinyl ether
HEMA = hydroxyethyl methacrylate
IPA = Isopropyl alcohol
MAA = methacrylic acid;
MMA = methyl methacrylate
mPDMS = mono-methacryloxypropyl terminated polydimethylsiloxane (MW 800-1000)
Norbloc = 2-(2'-hydroxy-5-methacrylyloxyethylphenyl)-2H-benzotriazole ppm = parts per million micrograms of sample per gram of dry lens PVP= polyvinylpyrrolidinone (K 90)
Simma 2 = 3-methacryloxy-2-hydroxypropyloxy)propylbis (trimethylsiloxy)methylsilane
TAA = t-amyl alcohol
TBACB = tetrabutyl ammonium-m-chlorobenzoate
TEGDMA = tetraethyleneglycol dimethacrylate
TEA = triethylamine
THF = tetrahydrofuran
TRIS = *tris*(trimethylsiloxy)-3-methacryloxypropylsilane
TMI = dimethyl meta-isopropenyl benzyl isocyanate
TMPTMA = trimethylolpropane trimethacrylate
w/w = weight/total weight
w/v = weightltotal volume
v/v =volume/total volume
3M3P = 3-methyl-3-pentanol.
The following compositions were prepared for use
Artifical Tears Solutions (ATS)

One liter of protein donor solution A (PD-A) consists of the following:
8.30g sodium chloride, Sigma
0.46g monobasic sodium phosphate, Sigma
4.40g dibasic sodium phosphate, Sigma
1.20g bovine plasma y-globulin, Sigma
1.20g chicken egg albumin, Sigma
1.20g chicken egg white lysozyme, Sigma

One liter of protein donor solution C (PD-C) consists of the following:
8.30g sodium chloride, Sigma
0.46g monobasic sodium phosphate, Sigma
4.40g dibasic sodium phosphate, Sigma
1.20g bovine plasma y-globulin, Sigma
0.03g bovine albumin, Sigma
1.20g chicken egg white lysozyme, Sigma

The ingredients are weighed out and placed in a 1000mL Erlenmeyer, which is then filled to the 1 L mark with deionized H₂O. The mixture is stirred with a magnetic stirrer at room temperature until all the components have dissolved and the solution becomes clear, typically for 15 to 30 minutes, and then transferred to plastic containers and stored in a refrigerator at 4°C throughout its use to prevent denaturing

### Macromer 2 Preparation

To a dry container housed in a dry box under nitrogen at ambient temperature was added 30.0 g (0.277 mol) of bis(dimethylamino)methylsilane, a solution of 13.75 mL of a 1M solution of TBACB (386.0 g TBACB in 1000 mL dry THF), 61.39 g (0.578 mol) of p-xylene, 154.28 g (1.541 mol) methyl methacrylate (1.4 equivalents relative to initiator), 1892.13 (9.352 mol) 2-(trimethylsiloxy)ethyl methacrylate (8.5 equivalents relative to initiator) and 4399.78 g (61.01 mol) of THF. To a dry, three-necked, round-bottomed flask equipped with a thermocouple and condenser, all connected to a nitrogen source, was charged the above mixture prepared in the dry box.

The reaction mixture was cooled to 15 °C while stirring and purging with nitrogen. After the solution reached 15 °C, 191.75 g (1.100 mol) of 1-trimethylsiloxy-1-methoxy-2-methylpropene (1 equivalent) was injected into the reaction vessel. The reaction was allowed to exotherm to approximately 62 °C and then 30 mL of a 0.40 M solution of 154.4 g TBACB in 11 mL of dry THF was metered in throughout the remainder of the reaction. After the temperature of reaction reached 30°C and the metering began, a solution of 467.56 g (2.311 mol) 2-(trimethylsiloxy)ethyl methacrylate (2.1 equivalents relative to the initiator), 3636.6. g (3.463 mol) n-butyl monomethacryloxypropyl-polydimethylsiloxane (3.2 equivalents relative to the initiator), 3673.84 g (8.689 mol), TRIS (7.9 equivalents relative to the initiator) and 20.0 g bis(dimethylamino)methylsilane was added.

The mixture was allowed to exotherm to approximately 38-42 °C and then allowed to cool to 30°C. At that time, a solution of 10.0 g (0.076 mol) bis(dimethylamino)methylsilane, 154.26 g (1.541 mol) methyl methacrylate (1.4 equivalents relative to the initiator) and 1892.13 g (9.352 mol) 2-trimethylsiloxy)ethyl methacrylate (8.5 equivalents relative to the initiator) was added and the mixture again allowed to exotherm to approximately 40°C. The reaction temperature dropped to approximately 30 °C and 2 gallons of THF were added to decrease the viscosity. A solution of 439.69 g water, 740.6 g methanol and 8.8 g (0.068 mol) dichloroacetic acid was added and the mixture refluxed for 4.5 hours to de-block the protecting groups on the HEMA. Volatiles were then removed and toluene added to aid in removal of the water until a vapor temperature of 110°C was reached.

The reaction flask was maintained at approximately 110°C and a solution of 443 g (2.201 mol) TMI and 5.7 g (0.010 mol) dibutyltin dilaurate were added. The mixture was reacted until the isocyanate peak was gone by IR. The toluene was evaporated under reduced pressure to yield an off-white, anhydrous, waxy reactive monomer. The macromer was placed into acetone at a weight basis of approximately 2:1 acetone to macromer. After 24 hrs, water was added to precipitate out the macromer and the macromer was filtered and dried using a vacuum oven between 45 and 60°C for 20-30 hrs.

### Macromer 5 Preparation

The following ingredients were combined using the steps below to generate Macromer 5.

| Chemical | Weight (g) or volume (ml) |
|---|---|
| bis(dimethylamino)-methylsilane | 5.72 g |
| 1.0 M Solution of tetrabutylammonium 3-chlorobenzoate (TBACB) in THF | 2.6 ml |
| p-xylene | 15.83 g |
| MMA | 29.44 g |
| Bloc-HEMA | 361.16 g |
| THF | 840 g |
| Methyltrimethylsilyl dimethylketene acetal | 38.53 g |
| 0.4 M solution of tetrabutylammonium 3-chlorobenzoate (TBACB) in THF | 6 ml |

| Step 2 | |
|---|---|
| Bloc-HEMA | 89.23 g |
| MPDMS | 693.00 g |
| TRIS | 701.46 g |
| bis(dimethylamino)-methylsilane | 3.81 g |

| Step 3 | |
|---|---|
| Solution of Bloc-HEMA | 361.16 g |
| MMA | 29.44 g |
| bis(dimethylamino)-methylsilane | 1.92 g |
| THF | 270 g |

| Step 4 | |
|---|---|
| Water | 93.9 g |
| Methanol | 141.2 g |
| Dichloroacetic acid | 1.68 g |

| Step 5 | |
|---|---|
| 3-Isopropenyl-α,α-dimethylbenzyl isocyanate | 169.07 g |
| TEA | 1.18 g |

Bloc-HEMA, MMA, mPDMS (about 800 to about 1000MW), TRIS, p-xylene and tetrahydrofuran (THF) were dried over preactivated 4A molecular sieve, and THF, mPDMS, and TRIS were passed through aluminum oxide column before use.

To a dry container in a dry box under nitrogen was added bis(dimethylamino)-methylsilane, a 1M solution of tetrabutylammonium 3-chlorobenzoate (TBACB) in THF, p-xylene, MMA (1.4 eqv. relative to initiator), Bloc-HEMA (8.5 eqv. relative to photoinitiator) and THF. The above mixture was charged to a dry flask equipped with a thermocouple and a condenser connected to a nitrogen source.

To the reaction mixture was injected methyltrimethylsilyl dimethylketene acetal while stirring and purging with nitrogen. The reaction was allowed to exotherm to about 65°C and then after the temperature of the solution dropped, a solution of TBACB in dry THF (0.4 M) was fed in slowly throughout the rest of the reaction. Then in step 2, a mixture of Bloc-HEMA (2.1 eqv. to initiator), mPDMS (3.3 eqv. to initiator), TRIS (7.9 eqv. to initiator) and bis(dimethylamino)-methylsilane, prepared in dry box, was added under nitrogen.

The reaction mixture was again allowed to exotherm to approximately 42°C and then allowed to cool to 32°C. The solution was stirred at 32°C by using a temperature controller and heating equipment for about five hours. In step 3, a mixture on of Bloc-HEMA (8.5 eqv. to initiator), MMA (1.4 eqv. relative to initiator) and bis(dimethylamino)-methylsilane was added and the whole mixture allowed to exotherm to 46 - 49°C. After the mixture reacted about two hours, 270 g of THF was added to reduce the viscosity and the solution was stirred for additional 30 minutes.

In step 4, a mixture of water, methanol and dichloroacetic acid was added and the mixture was refluxed for five hours to de-block the protecting groups. The solvents were then removed by distillation and toluene was added to aid in removal of residual water until a vapor temperature reached 110°C.

A solution of TMI and 1.2 mole % TEA relative to TMI was added to the above solution in toluene. The whole mixture was stirred at 110°C for three hours and the disappearance of the isocyanate peak was monitored by IR. The toluene was removed under reduced pressure at,around 45°C to give a raw macromer.

Purification procedures were employed to remove high molecular weight species. The raw macromer was re-dissolved in acetone (2:1 w / w acetone to macromer) and the acetone solution was set overnight to allow high molecular weight species to separate. The top clear phase was filtered through a PTFE membrane by pressure filtration. The filtrate was slowly charged into water (4:1 v / v water to filtrate) and the macromer was precipitated out. The macromer was collected and dried using a vacuum oven at 45 - 65°C under reduced pressure until there was no weight change.

Further purification to remove low molecular weight species was also done by re-precipitation of the macromer from the mixture of acetone and acetonitrile (1:5v/v).

### Packing Solution

Packing solution contains the following ingredients in deionized H₂O:
0.18 weight % sodium borate [*1330-43-4*], Mallinckrodt
0.91 weight % boric acid [*10043-35-3*], Mallinckrodt
0.83 weight % sodium chloride [7647 14-5], Sigma
0.01 weight % ethylenediaminetetraacetic acid [*60-00-04*] (EDTA), Aldrich

### Phosphate buffered saline (PBS)

PBS contains the following in deionized H₂O:
0.83 weight % sodium chloride [*7647-14-5*], Sigma
0.05 weight % monobasic sodium phosphate [*10049-21-5*], Sigma
0.44 weight % dibasic sodium phosphate [*7782-85-6*], Sigma

### Special packing solution SPS

SPS contains the following in deionized H₂O:
0.18 weight % sodium borate [*1330-43-4*], Mallinckrodt
0.91 weight % boric acid [*10043-35-3*], Mallinckrodt

### Lens Type A

A hydrogel blend was made from the following monomer mix (all amounts were calculated as weight percent: 17.98% Macromer 2, 28.0% mPDMS, 14.0% TRIS, 26.0% DMA, 5.0% HEMA, 1.0% TEGDMA, 5.0% PVP, 2.0% Norbloc, 1.0% CGI 1850 and 0.02% Blue HEMA, 80 weight percent of the preceding component mixture was further diluted with diluent, 20 weight percent of 30:70 dipropylene glycol:DPMA, to form the final monomer mix. Molds are coated with p-HEMA using the method disclosed in U.S. Pat App. No. 09/921,192, entitled "Method for Coating Articles by Mold Transfer," filed on August 2, 2001, before loading the blend to the molds of the type described in U.S. Patent 4,640,489. The front curve was coated with 5.8 cP and the back curve was coated with 4.5cP p-HEMA. Polymerization occurred under a nitrogen purge and was photoinitiated with visible light generated with four Philips TL 03 fluorescent bulbs (4 inches above the mold), at a temperatures of 50°C over 30 minutes.

### Lens Type B

Contact lenses prepared as described in U.S. Pat. App. No. 60/318,536, entitled "Biomedical Devices Containing Internal wetting Agents," filed on September 10, 2001 and its non-provisional counterpart of the same title, filed on September 6, 2002, containing by weight percent 28% SiMMA2, 31 % mPDMS, 23.5% DMA, 7% PVP (MW 360,000), 1.5%TEDGMA, 0.98% CGI 1850, 2.0% Norbloc, 6% HEMA, 0.02% Blue Hema. The aforementioned patents are hereby incorporated in reference in their entirety.

### p-HEMA

A homogeneous mixture of 615mg CGI 1850, 150mL of ethylene glycol, and 45g of hydroxyethyl methacrylate is degassed by evacuating the system for several minutes, followed by purging with nitrogen. This process is continued for one hour, using 3-4 evacuate/purge sequences, and the mixture is placed in a nitrogen atmosphere.

A solution of blue HEMA in ethylene glycol (0.9g in 200mL) is poured into the apparatus cover to provide the light filter required for this synthesis (1 08.75g of the solution for the described cover). This vessel is then placed on the Dewar-type flask, which has been charged with the reaction mixture. The junction of the reaction vessel and cover is tightly sealed using duct tape to avoid the penetration of any higher intensity light from the sides of the system.

The reaction is then exposed to visible light (Philips 20W/TLO3 bulbs) for one hour at room temperature, after which it is quenched by oxygen, and washed with D.I.water. The white polymer is washed several times with 100-150mL of water until its texture remains consistent and no additional hardening is observed. The rubbery material is then torn into smaller pieces, and stirred in 300mL of D.I. water for two hours. The liquids are decanted, and the water wash is repeated once more. The product is then squeeze dried, and placed in a rotary evaporator to remove the residual water at reduced pressure. The dried polymer is milled into fine particles prior to use.

GPC data for each of the polymers were obtained using both R.I and light scattering detectors. Chromatography was performed using a phenogel 5 microns linear (2) column (Phenomenex), and 0.5wt% lithium bromide in dimethylformamide as the eluent.

Mn = 2.876X106; Mw = 4.196X106, Mz = 6.548X106, polydispersity (Mw/Mn) = 1.459.

### Example 1

### Preparation of Lens Types 1-6

A hydrogel blend was made from the following monomer mix (all amounts were calculated as weight percent of the total weight of the components): 17.98% Macromer 2, 28.0% mPDMS, 14.0% TRIS, 26.0% DMA, 5.0% HEMA, 1.0% TEGDMA, 5.0% PVP, 2.0% Norbloc, and 0.02% Blue HEMA, 80 weight percent of the preceding component mixture was further diluted with diluent, 20 weight percent of D30. This blend was stirred at 50°C for 45 minutes and subsequently acetic acid (1% w/w) was added followed by an additional stirring (15 min.). Solid silver salts (purchased from Aldrich Chemical Company), were added in sufficient quantities to produce the target quantities listed in Table 1. A photoinitiator (CGI 1850 1.0 %) was added at the same time as the silver salt and the resulting mixture was stirred for an additional 45 minutes at 50°C, sonicated for 100 minutes at 50°C, degassed by vacuum for 15 minutes and placed on a roller at a minimum of 50 rpms overnight. This mixture was loaded to an eight cavity lens mold of the type described in U.S. Patent 4,640,489. Polymerization occurred under a nitrogen purge and was photoinitiated with visible light generated with four Philips TL 03 fluorescent bulbs (4 inches above the mold), at a temperatures of 50°C over 30 minutes. After curing, the molds were opened, and the lenses were pulled from the molds. A minimum number of three lenses from each set were dried in a vacuum oven for three to four hours at 80°C, at a maximum pressure of five inches of Hg, and sent to an independent laboratory for residual silver content measurements by instrumental neutron activation analysis (INAA).

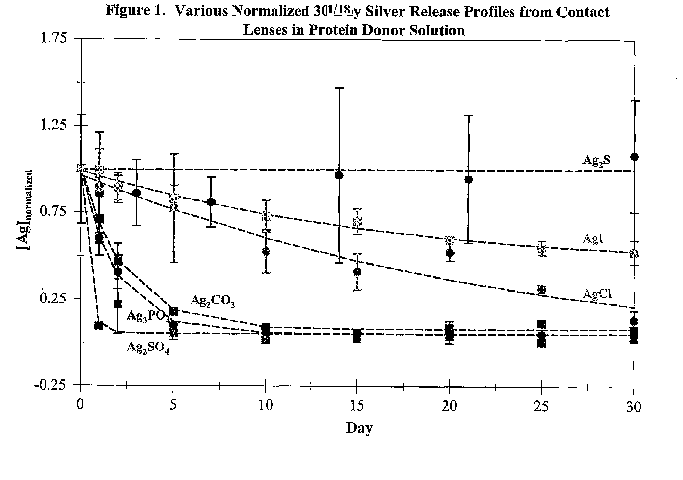

In order to obtain release profiles, the remaining lenses were separately placed in individual plastic vials with 2.2 mL standard PD-A which was exchanged every 24 hours. The vials were kept in a tray on a plate shaker throughout the experiment, at room temperature. Triplicate samples were pulled on different days throughout the thirty-day test period, dried as described previously, and sent to an independent laboratory for silver content analysis by INAA. Plots of some typical results (normalized when necessary to account for differences in initial silver concentration) are shown in Figure 1. The corresponding silver salt Kₛₚ, values and silver ion molar solubilities are listed in Table 1, along with the target, the initial (day 0) and final silver(day 30) concentration (ppm), as well as the time (in number of days) at which the silver concentration is ½ its initial concentration ("½ [Ag]") are listed in Table 1.

**Table 1**

| Lens Type | Silver Salt | Kₛₚ | Molar solubility, silver moles/L | Target Conc. ppm | [Ag] initial (n=3) ppm | [Ag] final (n=3) ppm | [Ag] in No. of days |
|---|---|---|---|---|---|---|---|
| 1 | Ag₂SO₄ | 1.20 x 10⁻⁵ | 2.88x 10⁻² | 500 | 237.2 ± 202.3 | 6.6 ±2.2 | 0.3 |
| 2 | Ag₂CO₃ | 8.45 x 10⁻¹² | 2.57x 10⁻⁴ | 500 | 960.9 ± 115.9 | 74.5 ±22.9 | 1.9 |
| 3 | Ag₃PO₄ | 8.88 x 10⁻¹⁷ | 1.68x 10⁻⁴ | 500 | 665.7± 56.2 | 34.0 ±8.0 | 1.4 |
| 4 | AgCl | 1.77 x 10⁻¹⁰ | 1.33x 10⁻⁵ | 750 | 790.1 ± 130.8 | 105.3 ±45.5 | 14 |
| 5 | Agl | 8.51 x 10⁻¹⁷ | 9.22 x 10⁻⁹ | 500 | 580.4. ± 130.8 | 305.7 ±38.8 | 36 |
| 6 | Ag₂S | ∼1,0 x 10⁻⁵⁰ | 2.71x 10⁻¹⁷ | 1000 | 1835.3 ± 578.3 | 1987.8±595.5 | >36 |

### Example 2

### Preparation of Lens Types 3-16, 50-52 and Release of Silver From Lens Types 9-16

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850 and 0.02% Blue HEMA, 80 weight percent of the preceding component mixture was further diluted with diluent, 20 weight percent of, 30:70 dipropylene glycol:DPMA. This blend shipped to a commercial miller of salts along with silver chloride and silver iodide purchased from Aldrich Chemical Company. The commercial miller, ground the silver salts to a mean particle size distribution of equal to or less than 10 microns and prepared solutions of the blend with predetermine amounts of milled silver chloride or silver iodide. Upon receipt, the resulting mixtures were rolled at 50 rpms until further use. The mixtures were loaded to an eight cavity lens mold of the type described in U.S. Patent 4,640,489. Polymerization occurred under a nitrogen purge and was photoinitiated with visible light generated with four Philips TL 03 fluorescent bulbs (4 inches above the mold), at a temperatures of 50°C over 30 minutes. After curing, the molds were opened, and the lenses were pulled from the molds. A minimum number of three lenses from each set were dried in a vacuum oven for three to four hours at 80°C, at a maximum pressure of five inches of Hg, and sent to an independent laboratory for residual silver content measurements by instrumental neutron activation analysis (INAA).

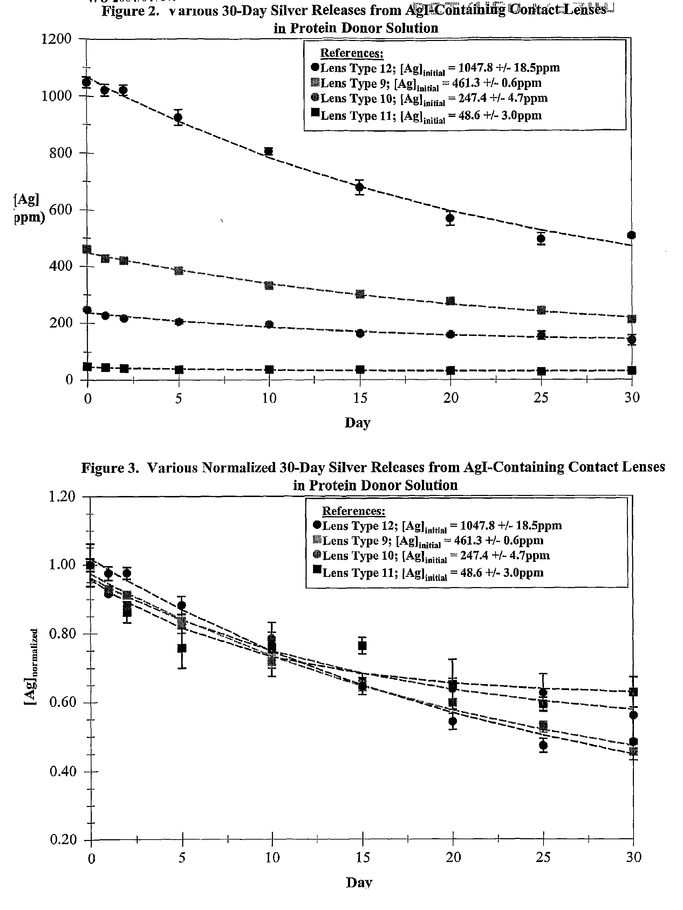

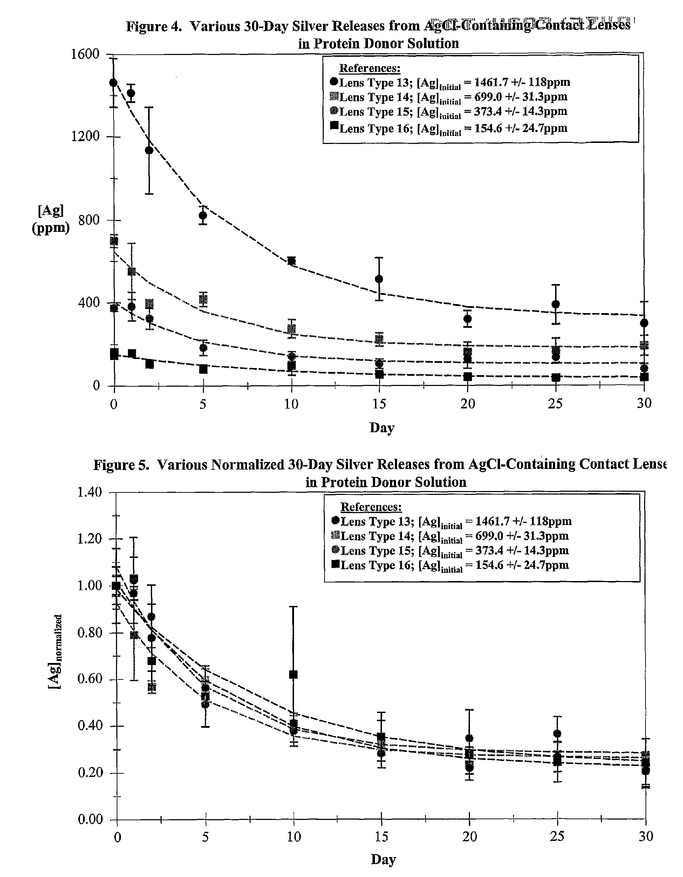

In order to obtain release profiles, the remaining lenses were placed in individual plastic vials with 2.2 mL PD-A which was exchanged every 24 hours. The vials were kept in a tray on a plate shaker throughout the experiment, at room temperature. Triplicate samples were pulled on different days throughout the thirty-day test period, dried as described previously, and sent to an independent laboratory for silver content analysis by INAA. Plots of the results are shown in Figure 2, 3, 4, and 5. Results for silver iodide are in Figures 2 and 3. Results for silver chloride are in Figures 4 and 5. The data in Figures 3 and 5 were normalized to account for differences in initial silver concentration . The corresponding, target and initial silver concentrations, are listed in Table 2 in ppm.

**Table 2**

| Lens Type # | Silver Salt | Target Conc. ppm | [Ag] initial (n=3) ppm |
|---|---|---|---|
| 9 | Agl | 1000 | 461.3 ± 0.6 |
| 10 | Agl | 500 | 247.4 ± 4.7 |
| 11 | Agl | 100 | 48.6± 3.0 |
| 12 | Agl | 1000 | 1047.8 ± 18.5 |
| 13 | AgCl | 1000 | 1461.7. ± 118.0 |
| 14 | AgCl | 500 | 669.03 ± 31.3 |
| 15 | AgCl | 250 | 373.4 ± 14.3 |
| 16 | AgCl | 100 | 154.6 ± 24.7 |

### Example 3

### Preparation of Lens Types 17 & 18 and Comparison of the Release Rates of Hydrated Lenses With Non-Hydrated Lenses

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850 and 0.02% Blue HEMA, 80 weight percent of the preceding component mixture was further diluted with diluent, 20 weight percent of, 30:70 dipropylene glycol:DPMA.. This blend shipped to a commercial miller of salts along with silver chloride and silver iodide purchased from Aldrich Chemical Company. The commercial miller, ground the silver salts to mean particle size distribution of equal to or less than 10 microns and prepared solutions of the blend with predetermine amounts of milled silver chloride or silver iodide. Upon receipt, the resulting mixtures were rolled at 50 rpms until further use. The mixtures were loaded to an eight cavity lens mold of the type described in U.S. Patent 4,640,489. Polymerization occurred under a nitrogen purge and was photoinitiated with visible light generated with four Philips TL 03 fluorescent bulbs (4 inches above the mold), at a temperatures of 50°C over 30 minutes. After curing, the molds were opened, and whichever curve the lenses were sticking to was placed in a 400mL jar. Up to 160 lenses (stuck to frames) were placed jar that were subsequently filled with a 40:60 mixture of deionized H₂O:HPLC grade 2-propanol (IPA) and left to sit overnight. The next day, the mold pieces were removed from the jar and the solution containing the demolded lenses was replaced with 400mL of 100% IPA and the jar placed on a roller at circa 30 rpms for a minimum of 30 minutes. The solution was subsequently changed to 20:80 H₂O:IPA, then 40:60 H₂O:IPA, 60:40 H₂O:IPA, 80:20 H₂O:IPA, to 100% deionized H₂O which was changed out three times. Between each solution change, the jar was placed on the roller for thirty minutes. The lenses were then moved into individual glass vials containing 3mL of either deionized H₂O or Packing Solution, sealed with stoppers and crimped foil caps, and then autoclaved for 30 minutes at 121°C. A minimum of three glass vials was decrimped, the lenses removed and dried as described previously, and sent to an independent laboratory for silver content analysis by INAA in order to obtain the hydrated silver values. The initial silver content of the silver iodide lenses of Lens Type 17 was 1213.3 ± 71.2 ppm. The initial silver content of the silver chloride lenses of Lens Type 18 was 1272.3 ± 177 ppm.

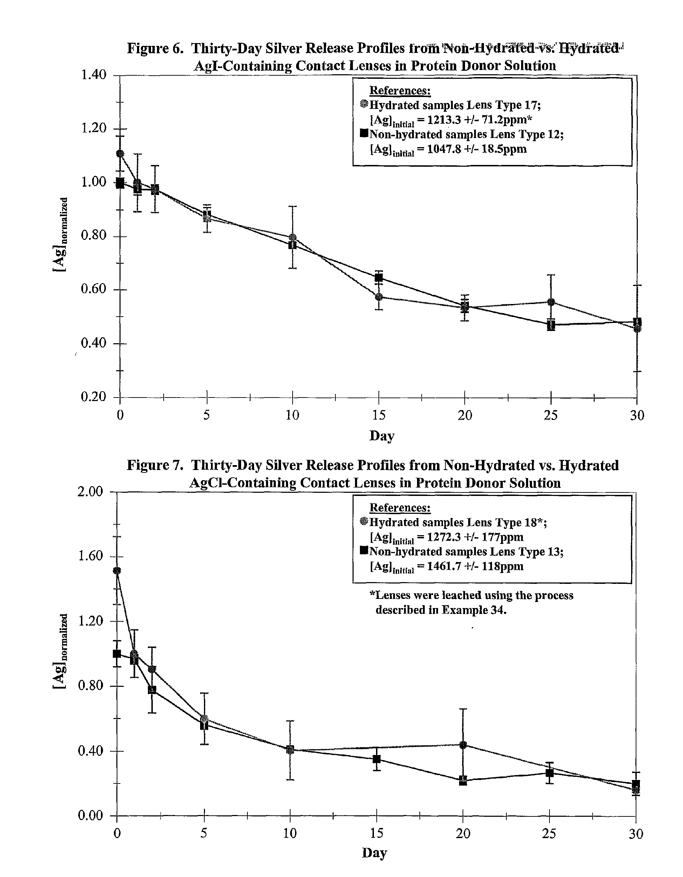

In order to obtain release profiles, the remaining lenses were placed in individual plastic vials with 2.2 mL PD-A which was exchanged every 24 hours. The vials were kept in a tray on a plate shaker throughout the experiment, at room temperature. Triplicate samples were pulled on different days throughout the thirty-day test period, dried as described previously, and sent to an independent laboratory for remaining silver content analysis by INAA. Plots of the results (normalized when necessary to account for differences in initial silver concentration) are shown in Figures 6 and 7. The results for Lens Type 17 are plotted with the results for the non-hydrated Lenses of Type 12. The results for Lens Type 18 are plotted with the results for the non-hydrated Lenses of Type 13. There were no noticeable differences in silver release behavior in protein donor solution. However, it should be noted that the release data of the hydrated examples were normalized to a value of one at the second time point (day one) and plotted against the profiles of the non-hydrated silver salt-containing lenses, which were normalized at the usual day zero.

### Example 4

### Preparation of Lens Types 19

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850 and 0.02% Blue HEMA, 80 weight percent of the preceding component mixture was further diluted with diluent, 20 weight percent of, 30:70 dipropylene glycol:DPMA. This blend shipped to a commercial miller of salts along with silver chloride purchased from Aldrich Chemical Company. The commercial miller, ground the silver salts to a mean particle size distribution equal to or less than 10 microns and prepared solutions of the blend with predetermined amounts of milled silver chloride. Upon receipt, the resulting mixtures were degassed in a vacuum dessicator for a minimum of 30 minutes and subsequently rolled at 50 rpms until further use. Molds are coated with p-HEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP p-HEMA. The lenses were cured under visible light Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3

### Example 5

### Preparation of Lens Types 20

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850 and 0.02% Blue HEMA, 80 weight percent of the preceding component mixture was further diluted with diluent, 20 weight percent of, 30:70 dipropylene glycol:DPMA. This blend shipped to a commercial miller of salts along with silver chloride purchased from Aldrich Chemical Company. The commercial miller, ground the silver salts to a mean particle size distribution equal to or less than 10 microns and prepared solutions of the blend with predetermine amounts of milled silver chloride. Upon receipt, the resulting mixtures were degassed in a vacuum dessicator for a minimum of 30 minutes and subsequently rolled at 50 rpms until further use. Molds are coated with p-HEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP p-HEMA. The lenses were cured under visible light Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses is listed in Table 3.

### Example 6

### Preparation of Lens Types 21

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. This blend shipped to a commercial miller of salts along with silver iodide purchased from Aldrich Chemical Company. The commercial miller, ground the silver salts to a mean particle size distribution equal to or less than 10 microns and prepared solutions of the blend with predetermine amounts of milled silver iodide. Upon receipt, the resulting mixtures were degassed in a vacuum dessicator for a minimum of 30 minutes and subsequently rolled at 50 rpms until further use. Molds are coated with p-HEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP p-HEMA. The lenses were cured under visible light Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3.

### Example 7

### Preparation of Lens Types 22

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. This blend shipped to a commercial miller of salts along with silver iodide purchased from Aldrich Chemical Company. The commercial miller, ground the silver salts to a mean particle size distribution equal to or less than 10 microns and prepared solutions of the blend with predetermine amounts of milled silver iodide. Upon receipt, the resulting mixtures were further diluted with the initial hydrogel blend without the silver salt (5%), degassed in a vacuum dessicator for a minimum of 30 minutes and subsequently rolled at 50 rpms until further use. Molds are coated with pHEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP pHEMA. The lenses were cured under visible light (Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3.

### Example 8

### Preparation of Lens Types 23

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. This blend shipped to a commercial miller of salts along with silver iodide purchased from Aldrich Chemical Company. The commercial miller, ground the silver salts to a mean particle size distribution equal to or less than 10 microns and prepared solutions of the blend with predetermine amounts of milled silver iodide. Upon receipt, the resulting mixtures were further diluted with the initial hydrogel blend without the silver salt (5%), degassed in a vacuum dessicator for a minimum of 30 minutes and subsequently rolled at 50 rpms until further use. Molds are coated with pHEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP pHEMA. The lenses were cured under visible light (Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3.

### Example 9

### Preparation of Lens Types 24

A hydrogel blend was made from the following monomer mix (all amounts were calculated as weight percent of the total weight of the components): 17.98% Macromer 5, 28.0% mPDMS, 14.0% TRIS, 26.0% DMA, 5.0% HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. This blend shipped to a commercial miller of salts along with silver iodide purchased from Aldrich Chemical Company. The commercial miller, ground the silver salts to a mean particle size distribution equal to or less than 10 microns and prepared solutions of the blend with predetermine amounts of milled silver iodide. Upon receipt, the resulting mixtures were further diluted with the initial hydrogel blend without the silver salt (50%), degassed in a vacuum dessicator for a minimum of 30 minutes and subsequently rolled at 50 rpms until further use. Molds are coated with pHEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP pHEMA. The lenses were cured under visible light Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3.

### Example 10

### Preparation of Lens Types 25

A hydrogel blend was made from the following monomer mix (all amounts were calculated as weight percent of the total weight of the components): 17.98% Macromer 5, 28.0% mPDMS, 14.0% TRIS, 26.0% DMA, 5.0% p-HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. This blend shipped to a commercial miller of salts along with silver iodide purchased from Aldrich Chemical Company. The commercial miller, ground the silver salts to a mean particle size distribution equal to or less than 10 microns and prepared solutions of the blend with predetermine amounts of milled silver iodide. Upon receipt, the resulting mixtures were further diluted with the initial hydrogel blend without the silver salt (5%), degassed in a vacuum dessicator for a minimum of 30 minutes and subsequently rolled at 50 rpms until further use. Molds are coated with pHEMA using the method disclosed in U.S. Pat App. No. 09/921,192, entitled "Method for Coating Articles by Mold Transfer." before loading the blend to the molds of the type described in U.S. Patent 4,640,489. The front curve was coated with 5.8 cP and the back curve was coated with 4.5cP p-HEMA. The lenses were cured under visible light Philips TL-03 bulbs in a nitrogen atmosphere (<0.5% O₂) for 12-15 minutes @ 70 ± 5°C. After curing, the molds were opened, and some of the lenses were pulled from the molds, dried as described previously, and sent to an independent laboratory for silver content analysis by INAA. The remaining lenses were released, leased and hydrated. The target silver concentration, the initial silver concentration of the non-hydrated lenses and the final silver concentration of the hydrated lenses are listed in Table 3 (numbers in parentheses are standard deviations).

**Table 3**

| Lens Type | Silver Type | Target [Ag] ppm | Non-hydrated [Ag] ppm | Hydrated [Ag] ppm | Percent Variability | % Ag Lost in Process |
|---|---|---|---|---|---|---|
| 22 | Agl | 50 | 120.7 (1.0) | 4.7(1.8) | 38.3 | 96.1 |
| 23 | Agl | 200 | N/A | 28.6(3.0) | 10.5 | N/A |
| 24 | Agl | 500 | 285.4 (9.4) | 134.8(7.6) | 5.6 | 52.8 |
| 25 | Agl | 1000 | 590.0 (28.4) | 377.1(30.4) | 8.1 | 36.1 |
| 21 | Agl | 500 | 408.7 (28.4) | 299.0 (23.3) | 9.5 | 26.8 |
| 20 | AgCl | 1000 | 173.2 (47.8) | 79.8 (68.1) | 85.3 | 53.9 |
| 19 | AgCl | 1000 | 212.1 (91.4) | 15.0(11.1) | 74.1 | 92.9 |

### Example 11

### Preparation and Testing of Lens Types 58-67

Lens Types 58-67 were made using the general method of Example 1, but substituting different silver salts and revising the demold, leach, hydration process as follows. The front and base curves of the plastic molds containing the cured lenses were separated manually and whichever curve the lens stuck to was placed in a 400mL glass jar. Each set of lenses, up to 20 frames or 160 lenses, was placed in their own jar, which was then filled with a 50:50 mixture of deionized H₂O:IPA (2-propanol) and left to sit overnight. The next day, the plastic curves were removed from the jar leaving behind only the lenses, the solution was replaced with 100% IPA, and the jar placed on a roller set to approximately 30 rpms and left to roll for a minimum of 30 minutes. The solution was subsequently replaced with a 50:50 mixture of deionized H₂O:IPA and then with 100% deionized H₂O which was changed out a total of three times. Between each solution change, the jar was placed on the roller for a minimum of 30 minutes. The initial efficacy was determined by the method of Example 12 and presented in Table 4.

**Table 4**

| **Silver Salt** | **Lens Type** | **Kₛₚ In pure H₂O at 25°C*** | **Molar Solubility (moles/L)** | **Hydrated Silver Content (ppm)** | **Log Reduction (Efficacy)**** |
|---|---|---|---|---|---|
| Silver lactate | 58 | N/A | N/A | 662.7(33.2) | 1.65 |
| Silver | 59 | | | | |
| acetylacetonate | | N/A | N/A | 2270.2(37.1) | 2.01 |
| Silver nitrate | 60 | | | | |
| [AgNO₃] | | 51.6 | 7.18 | 130.3(12.2) | 1.68 |
| Silver sulfate | 61 | | | | |
| [Ag₂SO₄] | | 1.20x10⁻⁵ | 2.89x10⁻² | 6.7(0.8) | 1.58 |
| Silver oxide | 62 | | | | |
| [Ag₂O] | | 7.06x10⁻¹³ | 1.12x10⁻⁴ | 4480.7(298.7) | 2.32 |
| Silver | 63 | | | | |
| phosphate | | | | | |
| [Ag₃PO₄] | | 8.88x10⁻¹⁷ | 1.28x10⁻⁴ | 77.8(10.9) | 2.09 |
| Silver iodate | 64 | | | | |
| [AglO₃] | | 3.17x10⁻⁸ | 1.78x10⁻⁴ | 296.3(55.7) | 1.89 |
| Silver | 65 | | | | |
| carbonate | | | | | |
| [Ag₂CO₃] | | 8.45x10⁻¹² | 2.57x10⁻⁴ | 232.0(78.4) | 1.88 |
| Silver bromide | 66 | | | | |
| [AgBr] | | 5.35x10⁻³ | 7.31x10⁻⁷ | 221.1(183.4) | 2.15 |
| Silver sulfide | 67 | | | | |
| [Ag₂S] | | ∼1.0x10⁻⁵⁰ | 2.71x10⁻⁷ | 1410.6(171.2) | 0.36 |

| | | | | | |
|---|---|---|---|---|---|
| *CRC Handbook of Chemistry and Physics, 74th edition **At 10⁶ cfu/mL inoculum vs. Lens Type A | | | | | |

### Example 12

### Initial Efficacy Determination

Lenses were placed in crimped glass vials (3mL volume in vial) or heat-sealed blister packages (950 µL volume in blister) with either Packing Solution, Special Packing Solution, or deionized water. The lenses were sealed (glass crimping or heat sealing) and sterilized by a 30 minute autoclave cycle at 121°C. Separately, a culture of *Pseudomonas aeruginosa*, ATCC# 15442 (American Type Culture Collection, Rockville, MD), was grown overnight in a tryptic soy medium. The culture was washed three (3) times in phosphate buffere saline (PBS, pH=7.4 ±0.2) and the bacterial pellet was resuspended in 10 mL of PBS. The bacterial inoculum was prepared to result in a final concentration of approximately 1 x 10⁶ colony forming units/mL (cfu/mL). Three sterilized contact lenses were rinsed in three changes of 30 mL of phosphate buffered saline (PBS, pH = 7.4 +/- 0.2) to remove residual solutions. Each rinsed contact lens was placed with 2 mL of the bacterial inoculum into a sterile glass vial, which was then rotated in a shaker-incubator (100 rpm) for two hours at 37 +/- 2°C. Each lens was removed from the glass vial, rinsed , five (5) times in three (3) changes of PBS to remove loosely bound cells, placed into individual wells of a 24-well microtiter plate containing 1 mL PBS, and rotated in a shaker-incubator for an additional 22 hours at 37 +/- 2°C. Each lens was again rinsed five (5) times with 3 changes of PBS to remove loosely bound cells, placed into 10 mL of PBS containing 0.05% (w/v) Tween^{™} 80, and vortexed at 2000 rpm for 3 minutes, employing centrifugal force to disrupt adhesion of the remaining bacteria to the lens. The resulting supernatant was enumerated for viable bacteria and the results of detectable viable bacteria attached to 3 lenses were averaged and this data is presented in Table 5 in the column entitled "Initial Efficacy," as the log reduction of the innoculum, as compared to control.

### Example 13

### Ex-Vivo Efficacy Determination

Lenses were placed in crimped glass vials (3mL volume in vial) or heat-sealed blister packages (950 µL volume in blister) with either Packing Solution, Special Packing Solution, or deionized water. The lenses were sealed (glass crimping or heat sealing) and sterilized by a 30 minute autoclave cycle at 121°C. Some of the lenses were worn by humans for a periods of time as listed in Table 5 ("HWCL"). The HWCL were placed in an empty, sterile plastic or glass vial and stored dry at -20°C directly after they are removed from the eye and until the bacterial adhesion experiments are performed. Separately, a culture of *Pseudomonas aeruginosa*, ATCC# 15442 (American Type Culture Collection, Rockville, MD), was grown overnight in a tryptic soy medium. The bacterial culture was washed three(3) times in PBS and resuspended in 10 mL of PBS. The bacterial inoculum was prepared to result in a final concentration of approximately 1 x 10³ colony forming units/mL (cfu/mL). Three non-worn control contact lenses were rinsed three times with 30 mL phosphate buffered saline (PBS, pH = 7.4 +/- 0.2) to remove residual packing solution. Each rinsed control lens and frozen HWCL was placed with two mL of the bacterial inoculum into a sterile glass vial, which was then rotated in a shaker-incubator (100 rpm) for two hours at 35 +/- 2°C. Each lens was removed from the glass vial, rinsed five times with three changes of PBS to remove loosely bound cells, placed into individual wells of a 24-well microtiter plate containing 1 mL PBS, and rotated in a shaker-incubator for an additional 22 hours at 35 +/- 2°C. After incubation, each lens was again rinsed five times in three changes of PBS to remove loosely bound cells, placed into 10 mL of PBS containing 0.05% (w/v) Tween^{™} 80, and vortexed at 2000 rpm for three minutes. The resulting supernatant was enumerated for viable bacteria and the results of detectable viable bacteria attached to three lenses were averaged and the data is displayed in Table 5 in the column entitled "Ex-Vivo Efficacy," as the log reduction of the innoculum, as compared to control.

### Example 14

### Thirty Day Efficacy in the Presence of Artifical Tears Solution

Microtiter plates for lens incubation were prepared by placing 500µL of artificial tears solution, PD-C in each well of a 24-well microtiter plate. The test lenses were rinsed in three changes of PBS (30 mL) to remove residual packing solution and transferred aseptically into individual wells of each set of microtiter plates. The microtiter plates were then placed on an orbital shaker and allowed to incubate for 24 hours at room temperature. After incubation, lenses were either transferred into the wells of new microtiter plates containing 500µL ATS as described above or removed for bacterial challenge as described in Example 12. The data for this test is displayed in Table 5 in the column entitled "Protein Efficacy," as the log reduction of the innoculum, as compared to control, a comparable lens without silver salts. In the Protein Efficacy column, the log reduction was measured on particular day. For example the designation 3.04/PD-A indicates that on day 8 there was a 3.04 log reduction as measured after incubation in PD-A.

**Table 5**

| **Silver Type** | **Lens Type Hydrated [Ag] (ppm)** | **Initial Efficacy** | **Protein Efficacy** | **Ex- Vivo Efficacy** |
|---|---|---|---|---|
| Agl | 9 *¹ 472.5(7.9) | 1.77 | N/A | N/A |
| Agl | 9 *¹ 407.1(27) | 1.95 | N/A | N/A |
| Agl | 10 *¹ 250.1 (9.3) | 1.65 | N/A | N/A |
| Agl | 11 *¹ 41.8(2.4) | 1.84 | N/A | N/A |
| Agl | 50 *¹ 215.2(23) | 1.89 | N/A | N/A |
| | | | 3.04/PD-A day 8, | |
| Agl | 51 *¹ 767.4(20) | 1.61 | 3.53/PD-C day 8 | N/A |
| | | | 2.72/PD-A day 8, | |
| AgCl | 52 *¹ 590.9(133) | N/A | 3.09/PD-C day 8 | N/A |
| Agl | 25 377.1(30) | 2.19 | [4.43/PD-C day 7 | N/A |
| | | | 5.52 day1, 4.77 day2 | |
| | | | 4.02 day10, 3.98 day15 | |
| | | | 3.64 day18, 3.42 day25 | |
| | | | 3.60 day30/PD-C] | |
| Agl | 24 134.8(7.6) | 2.01 | N/A | N/A |
| Agl | 23 28.6(3.0) | 2.32 | N/A | N/A |
| Agl | 22 4.7(1.8) | 1.97 | N/A | N/A |
| Agl | 21 299.0(28) | 1.63 | N/A | 1.90, 1.01 9hrs |
| | | | | 1.41,1.32 day3 |
| | | | | 0.81, 0.97 day7 |
| AgCl | 20 79.8(68) | 0.98 | 1.04/PD-C day 7 | N/A |
| | | | 5.52 day1, 2.42 day3 | |
| | | | 2.07 day10, 1.27 day 15 | |
| | | | 1.17 day18, 0.58 day25 | |
| | | | 0.53 day30 | |
| AgCl | 19 22.4(22) | 1.25 | N/A | N/A |

| | | | | |
|---|---|---|---|---|
| *¹ Lenses were leached using the process described in Example 34. | | | | |

### Example 15

### Preparation and Silver Analysis of Lens Type 33

Sodium Iodide (71.5 mg) was added to a hydrogel blend was made from the following monomer mix (all amounts were calculated as weight percent of the total weight of the combination): 17.98% Macromer 2, 28.0% mPDMS, 14.0% TRIS, 26.0% DMA, 5.0% HEMA, 1.0% TEGDMA, 5.0% PVP, 2.0% Norbloc, and 0.02% Blue HEMA, 80 weight percent of the preceding component mixture was further diluted with diluent, 20 weight percent of D30. The mixture was sonicated for 2.5 hours, and then stored in a heated oven @ 55°C overnight. The next day acetic acid and CGI 1850 (Ciba) were added in quantity sufficient to yield final concentrations of 1 w/w% and 0.8 w/w%, respectively. The monomer mix was degassed under vacuum for 30 minutes, and used to make lenses, utilizing Topas (Ticona, grade 5013) at 50-55°C under Philips TL03 lamps with 30 minutes of irradiation.

The lenses were manually demolded, and ten lenses (obtained sticking to the back curve or front curve) were immersed in a jar containing 80 mL of silver nitrate solution (0.228 mg/mL in DI water). The lenses were rolled on a jar roller for 2 hours, and then 120 mL of isopropanol was added to release the lenses from the molds.

The released lenses were transferred into 100 mL of IPA, and then stepped down into DI water as follows: i) 50 mL of 75:25 (IPA:DI water); ii) 50 mL of 50:50 (IPA:DI water); iii) 50 mL of 25:75 (IPA:DI water); iv) 30 mL of DI water; v) 100 mL of DI water; vi) 50 mL of DI water; iv) 50 mL of DI water; iv) 50 mL of DI water. Lenses from the last DI water wash were stored in a 10 mL of fresh DI water. The lenses were autoclaved (each in 3.0 mL of Special Packing Solution). The silver content of lenses was evaluated using the methods of Example 1. The averages were obtained from a sample size of 3 to 5 lenses to give a final silver content of Lens Type 33 of 3799 ± 160 ppm.

The following sample calculation provides an estimate of the maximum *theoretical* amount of silver per lens:
71.5 mg of Nal were dissolved in 15.0 g of reactive monomer mix (i.e. 4.77 mg/gram reactive monomer mix or 0.477% w/w).
Reactive monomer mix contained 20% D₃O (3,7-dimethyl-3-octanol or tetrahydrolinalool) as diluent.
Then, moles of Nal per gram of monomer mix => 0.00477g/149.89 g per mole = 3.18e-5 mol
Moles of silver ion per gram of monomer mix, assuming quantitative yield = 3.18e-5 mol
Thus amount of silver content per gram of monomer mix => 3.18e-5 mol x 107.868 g /mol = 0.00343 g
Since reactive monomer mix is 20% D₃O, the upper limit on silver content per gram of lenses => 0.00343 g / 0.8 = 0.00429 g

Thus the maximum theoretical concentration of silver per lens, ignoring all hydration losses is 0.4290% or 4290 ppm/

The average silver content of the lenses prepared in Example 15 was close to the theoretical maximum. The haze is measured by placing a hydrated test lens in borate buffered saline in a clear 20 x 40 x 10 mm glass cell at ambient temperature above a flat black background, illuminating from below with a fiber optic lamp (Titan Tool Supply Co. fiber optic light with 0.5" diameter light guide set at a power setting of 4-5.4) at an angle 66° normal to the lens cell, and capturing an image of the lens from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The subtracted scattered light image is quantitatively analyzed, by integrating over the central 10 mm of the lens, and then comparing to a CSI Thin Lens®, (CSI Flexible Wear (crotofilcon A) lot ML 62900207 Power -1.0) which is arbitrarily set at a haze value of 100, with no lens set having a haze value of 0. Four lenses are analyzed and the results are averaged to generate a haze value as a percentage of the standard CSI lens. The average haze value for these lenses (non-autoclaved) was 208.5 ± 25% relative to CSI commercial standards. Furthermore, examination of one of these lenses by optical and scanning electron microscopy, at magnifications ranging from 40X to 20,000X, provided no evidence of silver-containing particles (>200 nm) on the lens surface. Since the instrument cannot detect particles of a size lower than 200nm, all silver particles in the lens are presumed to be less than 200 nm.

### Example 16

### Preparation and Silver Analysis of Lens Type 34

Sodium Iodide (7.3 mg, Aldrich) was added to a hydrogel blend made from the following (all amounts were calculated as weight percent of the total weight of the combination): 17.98% Macromer 5, 28.0% mPDMS, 14.0% TRIS, 26.0% DMA, 5.0% HEMA, 1.0% TEGDMA, 5.0% PVP, 2.0% Norbloc,1.0% CGI 1850 and 0.02% Blue HEMA, 80 weight percent of the preceding component mixture was further diluted with diluent, 20 weight percent of D3O (hereinafter "D30 Monomer Blend"). The mixture was sonicated for 1.5 hours at 30°C, and then stored in a heated oven @ 55°C overnight. The monomer mix was degassed under vacuum for 30 minutes, and used to make lenses, utilizing Topas frames at 50-55°C under Philips TL03 lamps with 30 minutes of irradiation.

Eight frames of lenses were manually demolded, and (obtained sticking to the back curve or front curve) were immersed in a jar containing 300 mL of silver nitrate solution (0.15 mg/mL in DI water). The lenses were rolled on a jar roller for 2 hours, and then 180 mL of solution were replaced with 180 mL isopropanol to release the lenses from the molds.

After 2 hours, the released lenses were transferred into 200 mL of IPA, and then stepped down into DI water by (3 x 40 mL) exchanges of the hydration solution with DI water, allowing the lenses to equilibrate ½ hour between exchanges. The lenses were then transferred into DI water for a total of 4 x 75 mL washes of 20-30 minutes each. Lenses from the last DI water wash were stored in a 10 mL of fresh DI water. The lenses were autoclaved (each in 3.0 mL of special packing solution i.e. borate buffer). The final silver content of the lenses was 526 ± 21 ppm (theoretical = 436 ppm), as measured in Example 15. The average haze value for these lenses (non-autoclaved) was 17.3 ± 3% relative to CSI commercial standards.

### Example 17

### Preparation and Silver Analysis of Lens Type 35

Tetrabutylammonium chloride (17.6 mg, Fluka) was added to 15.1 g of D3O Monomer Blend. The mixture was sonicated for 1 hour, rolled on a jar roller for a further 30 minutes, and then stored in a heated oven @ 55°C overnight. The monomer mix was degassed under vacuum for 30 minutes, and used to make lenses, utilizing Topas frames at 50°C under Philips TL03 lamps with 30 minutes of irradiation.

Ten frames of lenses were manually demolded, and the rings due to excess monomer were discarded. The lenses, obtained sticking to either the back curve or front curve, were immersed in a jar containing 300 mL of silver nitrate solution (0.155 mg/mL in DI water). The lenses were rolled on a jar roller for 2 hours, and then 180 mL of solution were replaced with 180 mL isopropanol to release the lenses from the molds.

The released lenses were then further hydrated analogously to the procedure described in Example 16. The final silver content of Lens Type 35 was 584 ± 19 ppm (theoretical = 564 ppm), as measured by the methods of Example 15. The average haze value for these lenses was 53.6 ± 12.5% relative to CSI commercial standards.

### Example 18

### Preparation and Silver Analysis of Lens Type 36

Tetrabutylammonium Chloride (8.9 mg, Fluka) was added to 15.1 g of D3O Monomer Blend. The mixture was sonicated for 1 hour, rolled on a jar roller for a further 30 minutes, and then stored in a heated oven @ 55°C overnight. The monomer mix was degassed under vacuum for 30 minutes, and used to make lenses, utilizing Topas frames at 50°C under Philips TL03 lamps with 30 minutes of irradiation.

Ten frames of lenses were manually demolded, and the rings due to excess monomer were discarded. The lenses, obtained sticking to either the back curve or front curve, were loaded onto hydration trays, which were then immersed in 2.75 L of silver nitrate solution ('silverizing bath', 0.155 mg/mL in DI water). The silver nitrate solution was kept agitated by utilizing an orbital shaker. After 2 hours, the lenses were removed from the hydration trays, and placed in a jar containing 120 mL solution from the 'silverizing bath' and 180 mL isopropanol to release the lenses from the molds.

The released lenses were then further hydrated analogously to the procedure described in Example 16. The final silver content in the lenses was determined to be 331 ± 7 ppm (theoretical = 286 ppm), as measured by the methods of Example 15. The average haze value for these lenses was 21.8 ± 1.8% relative to CSI commercial standards.

### Example 19

### Preparation and Silver Analysis of Lens Type 37

Tetrabutylammonium bromide (20.3 mg, Fluka) was added to 15.0 g of D3) Monomer Blend. The mixture was sonicated for 2 hours. The monomer mix was degassed under vacuum for 5 minutes, and used to make lenses, utilizing Topas frames at 50°C under Philips TL03 lamps with 30 minutes of irradiation.

The lenses were manually demolded, and the rings due to excess monomer were discarded. The lenses, obtained sticking to either the back curve or front curve, were immersed in a jar containing 300 mL of silver nitrate solution (0.15 mg/mL in DI water). After 2 hours, the lenses were released from the molds and hydrated analogously to the procedure described in Example 16.The final silver content in the lenses was 686 ± 81 ppm (theoretical = 566 ppm), as measured by the methods of Example 15. The average haze value for these lenses was 63.5 ± 8.4% relative to CSI commercial standards.

### Example 20

### Preparation and Silver Analysis of Lens Type 38

Tetrabutylammonium chloride (13.0 mg, Fluka) was added to 21.77 g of D3O Monomer Blend. The mixture was sonicated for 1.5 hours. The monomer mix was degassed under vacuum for 10 minutes, and used to make lenses, utilizing Topas frames at 50-55°C under Philips TL03 lamps with 40 minutes of irradiation.

Five frames of lenses were manually demolded, and (obtained sticking to the back curve or front curve) were immersed in a jar containing 200 mL of silver nitrate solution (0.155 mg/mL in DI water). The lenses were rolled on a jar roller for 2 hours, and then transferred to 200 mL of 60:40 isopropanol: DI water, to release the lenses from the molds.

After 1 hour, the released lenses were transferred into 200 mL of IPA, and then stepped down into DI water by (4 x 40 mL) exchanges of the hydration solution with DI water, allowing the lenses to equilibrate 20 minutes between exchanges. The lenses were then transferred into DI water for a total of 3 x 200 mL washes of 20 minutes each. The final silver content of Lens Type 38 was 273 ± 15 ppm (theoretical = 290 ppm), as measured by the methods of Example 15.

### Example 21

### Preparation and Silver Analysis of Lens Type 39

Tetrabutylammonium chloride (13.0 g, Fluka) was added to 21.77 g of D3O Monomer Blend. The mixture was sonicated for 1.5 hours. The monomer mix was degassed under vacuum for 10 minutes, and used to make lenses, utilizing Topas FC BC frames at 50°C under Philips TL03 lamps with 40 minutes of irradiation.

Five frames were manually demolded, and the lenses (obtained sticking to the back curve or front curve) were enclosed individually in hydration vehicles (Fisher brand, HistoPrep disposable plastic tissue capsules) and immersed in 600 mL 90:10 DPM:DI water solution, at 55°C, containing 30 mg of silver nitrate and 45 mg silver triflate. An orbital shaker was used to keep the solution agitated. After 1.5 hours, the lenses were transferred in to 400 mL DI water at 45°C. This step was repeated two more times, allowing 30 minutes per wash. The final silver content in the lenses was determined to be 249 ± 27 ppm (theoretical = 290 ppm) by the methods of Example 15.

### Example 22

### Preparation and Silver Analysis of Lens Type 40

Sodium Iodide (14.7 mg, Aldrich) was added to 30.0 g of D3O Monomer Blend. The mixture was sonicated for 3 hours at 40-50°C, and then stored in a heated oven @ 55°C overnight. The monomer mix was degassed under vacuum for 10 minutes, and used to make lenses, utilizing Topas frames at 50-55°C under Philips TL03 lamps with 30 minutes of irradiation.

Four frames of lenses were manually demolded. The lenses, obtained sticking to either the back curve or front curve, were loaded onto hydration trays, which were then immersed in 3 L (60:40) IPA:DI water solution containing 100 ppm of silver nitrate solution ('silverizing bath'). The silver nitrate solution was kept agitated by utilizing an orbital shaker. After 1 hour and 15 minutes, the lenses were removed from the hydration trays, and placed in a jar containing 200 mL of isopropanol. The lenses were rolled on a jar-roller for 15 minutes, and then transferred into 200 mL of 80:20 IPA:DI water. The lenses were kept rolling on a jar-roller, and every 15 minutes, 40 mL of the solution was exchanged out with DI water. This step was repeated a total of four times, and then the lenses were transferred into 200 mL DI water. The lenses were rolled on a jar-roller for three days, and then washed two more times with 200 mL of DI water, allowing 2 hours per wash on the jar-roller. The final silver content in the lenses was determined to be 421 ± 33 ppm (theoretical = 441 ppm) by the methods of Example 15.

### Example 23

### Preparation and Silver Analysis of Lens Type 41

Sodium Iodide (0.38 g, Aldrich) was dissolved in 12.25g DMA. This mixture was added to the following components of the monomer blend, 122.36g Macromer 5, 190.53g mPDMS 1000, 95.27g TRIS, 164.68g DMA, 34.04g HEMA, 6.80g TEGDMA, 13.62g Norbloc, 0.14g Blue HEMA, 34.01g PVP, 6.81g CGI 1850, and 170.14g D₃O as the diluent. This monomer mix was degassed at 40 mmHg at a temperature of 55°C for a total of 30 minutes. The monomer mix was used to prepare lenses using Zeonor (Zeon, grade 1060R) front curves, and Polypropylene (Fina, grade EOD 00-11) back curves. The molds had been previously spin-coated with p-HEMA, in order to provide a mold transfer coating to the lenses, using the methods disclosed in WO03/11551. The lenses were cured under visible light (Philips TL-03 bulbs in a nitrogen atmosphere (<0.5% O₂) for 12-15 minutes @ 70 ± 5 °C.

The cured lenses were demolded, and loaded into hydration trays (32 lenses per tray), which were then immersed in a ∼100 ppm silver nitrate in DI water solution (∼100 L) for 2 hours. The hydration trays were then transferred into 60:40 IPA:DI water for 1.5 hours to release the lenses from the mold (back curve). The lenses were then swabbed into jars containing IPA. The lenses were rolled on a jar roller, and the IPA was exchanged out four times, allowing 2 hours in between exchanges. The lenses were then stepped down from neat IPA into DI water, by exchanging out: a) 10% of the IPA for DI water; b) 20% of the solution for DI water; c) 30% of the solution for DI water; d) 40% of the solution for DI water; e) 50% of the solution for DI water; f) 75% of the solution for DI water; g) 100% of the solution for DI water; h) 100% of the solution for DI water; i) 100% of the solution for DI water. The exchanges were performed at 20-minute intervals. The lenses were autoclaved in 3 mL of special packing solution. [Ag] Target: 400 ppm; observed 353 ± 22 ppm, as measured by the methods of Example 15. The average haze value for the lenses was 84.3 ± 3.1% relative to CSI commercial standards.

### Example 24

### Preparation of Lens Type 26

Sodium Iodide (3.2 mg Aldrich) was added to 15.0 g of D3O Monomer Blend. The mixture was sonicated for 1.5 hours at 30°C, and then stored in a heated oven @ 55°C overnight. The monomer mix was degassed under vacuum for 30 minutes, and used to make lenses, utilizing Topas frames at 50-55°C under Philips TL03 lamps with 30 minutes of irradiation.

Eight frames were manually demolded, and the lenses (obtained sticking to the back curve or front curve) were enclosed individually in hydration vehicles (Fisher brand, HistoPrep disposable plastic tissue capsules) and immersed in 2L of DI water containing 447 mg AgNO₃. An orbital shaker was used to keep the solution agitated. After 2 hours, the lenses were removed from the hydration vehicles, and released from the molds in a jar containing 150 mL of silver nitrate solution (0.15 mg/mL in DI water) and 225 mL isopropyl alcohol. After 2 hours, the solution was replaced with 200 mL isopropanol, and the lenses were then stepped down into DI water by (4 x 40 mL) exchanges of the hydration solution with DI water, allowing the lenses to equilibrate 20-30 minutes between exchanges. The lenses were then transferred into DI water for a total of 4 x 75 mL washes of 20-30 minutes each. Lenses from the last DI water wash were stored in a 10 mL of fresh DI water. The lenses were autoclaved (each in 3.0 mL of Special Packing Solution i.e. borate buffer). The final silver content in the lenses was determined by the methods of Example 1 to be 286 ± 15 ppm (theoretical = 189 ppm).

### Example 25

### Evaluation of Autoclave Stability of Packaging Solutions

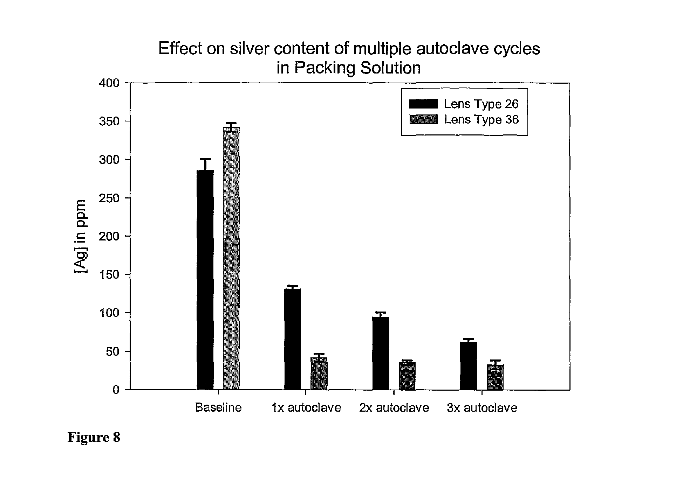

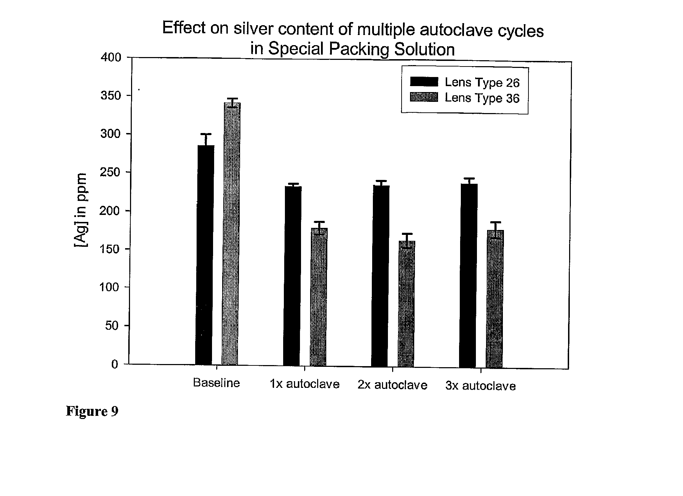

Lens Type 36 and Lens Type 26 were packaged in 3.0 mL glass vials half of the lenses of each lens type were packaged in Packing Solution and the other half were packaged in Special Packing Solution and sealed. The lenses were autoclaved one to three times at 121°C for 30 minutes. The lenses were evaluated for silver content using the methods of Example 15 and the data is presented in Figures 8 and 9. This experiment illustrates that more silver is retained in lenses that are autoclaved in Special Packing Solution.

### Example 26

### Preparation of Lens Types Evaluation of Silverizing Solutions

Lens Types 42-44 were prepared using the method of Example 20 and substituting tetrabutylammonium chloride with the amounts of sodium iodide displayed in Table 6 and two different concentrations of silverizing solution (10 ppm silver nitrate, 100 ppm silver nitrate). The theoretical amount of silver and the observed amounts of silver were determined using the methods of Example 15. The data is displayed in Table 6.

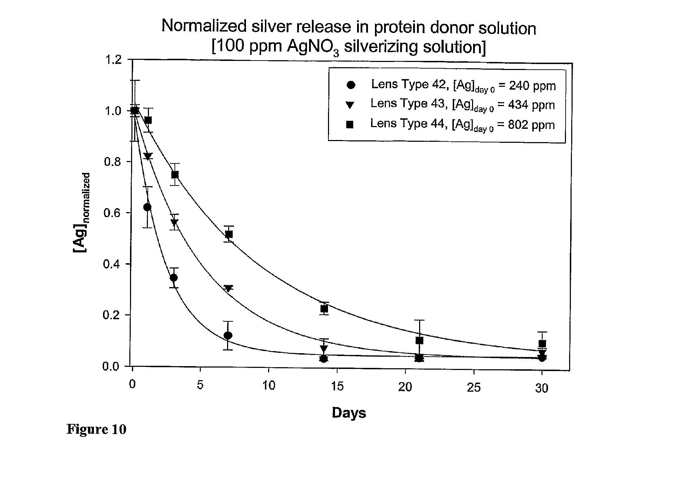

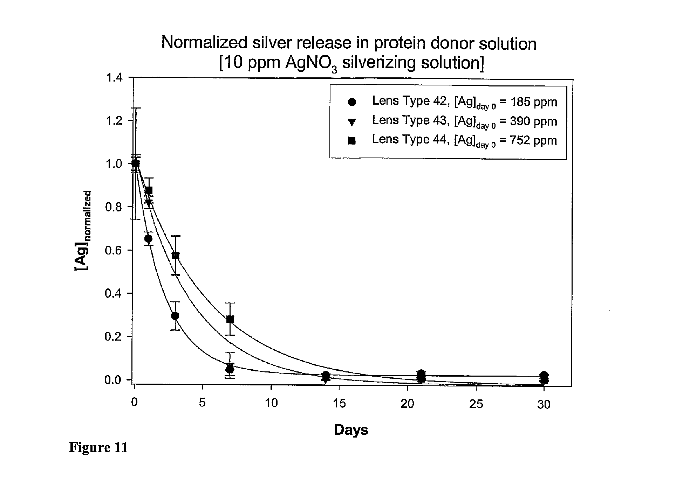

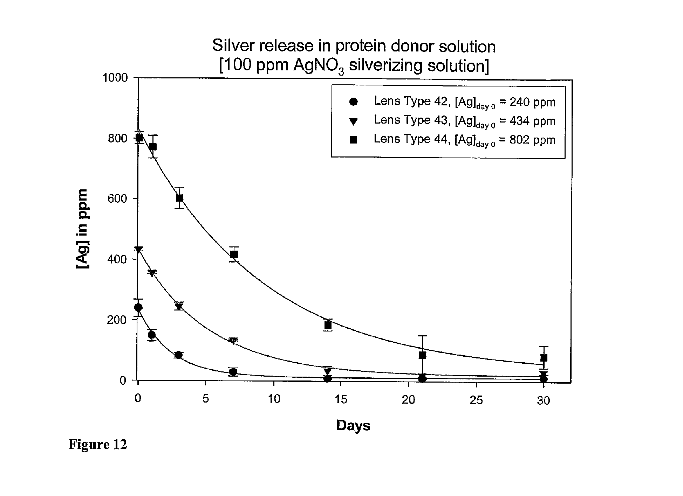

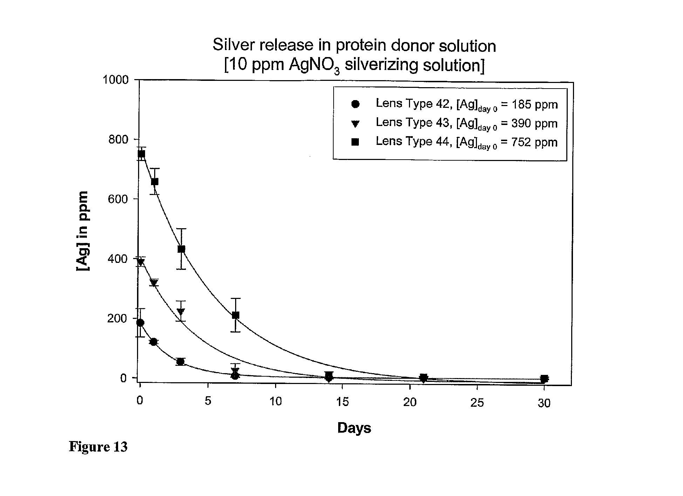

Subsequently the lenses were evaluated for their silver release profiles using the methods of Example 1 The data is displayed in Figures 10, 11, 12, and 13. This data illustrates that lenses treated with 100 ppm silver nitrate on the average, release silver slower at higher silver concentrations.

**Table 6**

| Lens Type | Amount of Nal in monomer mix | Theoretical [Ag] | Observed [Ag], 100 ppm AgNO₃ silverizing bath | Observed [Ag], 10 ppm AgNO₃ silverizing bath |
|---|---|---|---|---|
| 42 | 209 ppm | 188 ppm | 240 +/- 28.7 ppm | 185 +/-47.5 ppm |
| 43 | 410 ppm | 369 ppm | 434 +/- 4.63 ppm | 390 +/- 16.2 ppm |
| 44 | 812 ppm | 730 ppm | 802 +/-19.1 ppm | 752 +/- 22.7 ppm |

### Example 27

### Evaluation of Silverizing Time

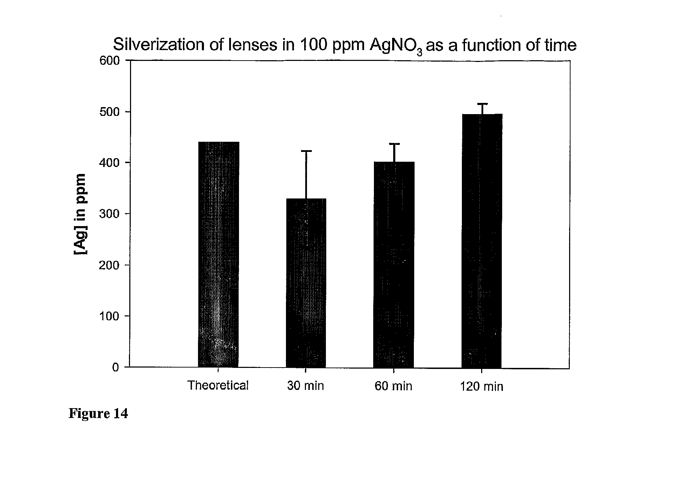

Lenses prepared by the method of Example 20, substituting Nal (14.7 mg) for tetrabutylammonium chloride and 100ppm silver nitrate (0.1 mg/mL). The amount of time that the lenses are stirred with the silverizing solution is varied and the amount of silver in the lenses is calculated using the method of Example 15. The data is displayed in Figure 14. This data shows that two hours of stirring produces lenses that have a silver content close to the theoretical target and the lowest standard deviation.

### Example 28

### Evaluation of Release Profiles of Different Counter Ions

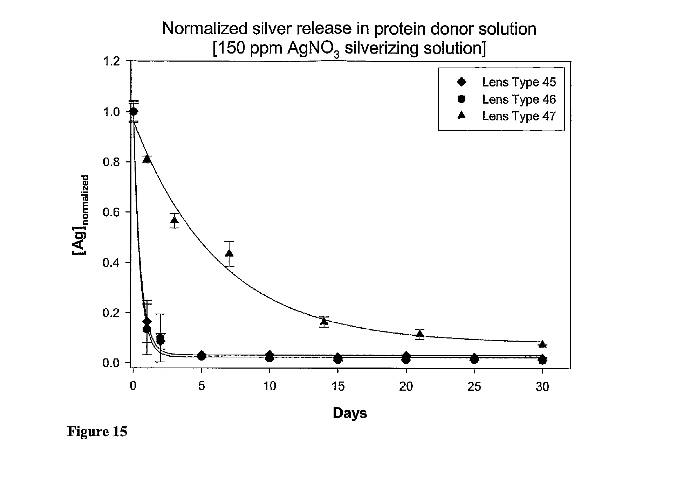

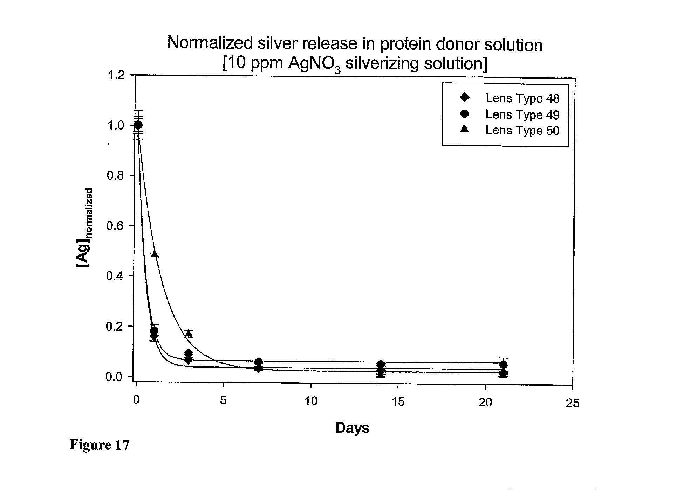

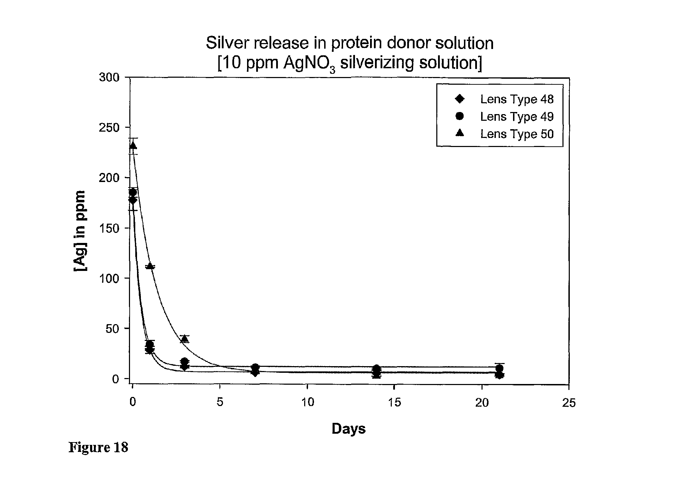

Lens Types 45 (containing AgCl), 46 (containing AgBr), and 47 (containing Agl) were prepared using the method of Example 16, 150 ppm silver nitrate silvering solution and the following anion salts tetrabutylammonium chloride, tetrabutylammonium bromide, and sodium iodide, respectively. Lens Types 48 (containing AgCl), 49 (containing AgBr), and 50 (containing Agl) were prepared using the method of Example 16, 10 ppm silver nitrate silvering solution and the following anion salts tetrabutylammonium chloride, tetrabutylammonium bromide, and sodium iodide, respectively. The silver release profiles were determined using the method of Example 1 and displayed in Figures 15, 16, 17, and 18. This data illustrates that Agl lenses release slower than AgCl or AgBr lenses.

### Example 29

### Evaluation of Release Profiles of Coated Lenses

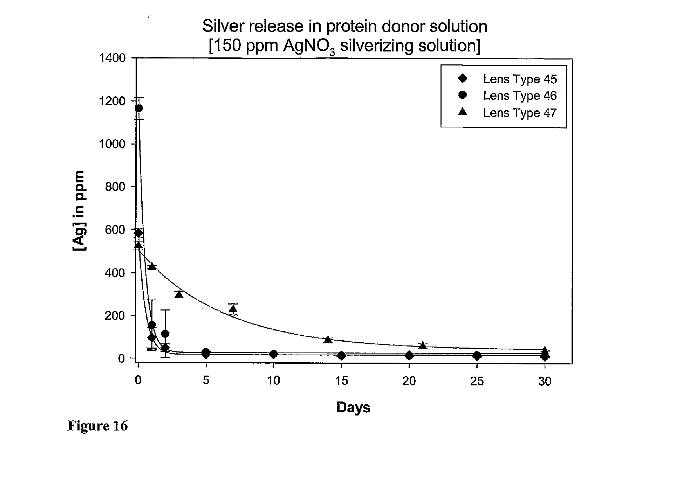

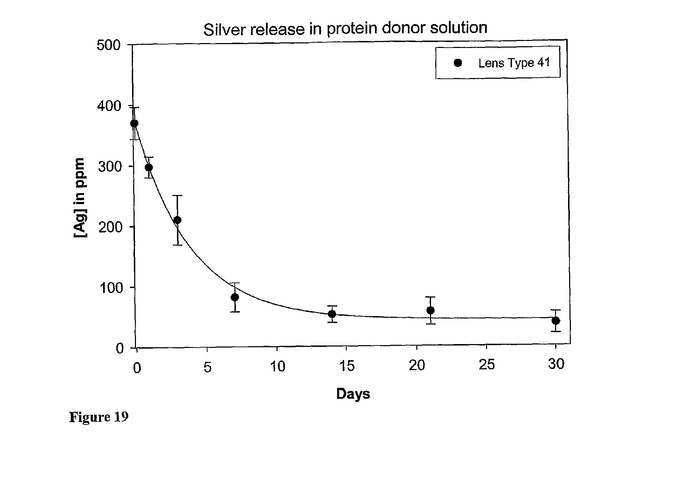

The release profiles of Lens Type 41 were evaluated by the method of Example 1 and displayed in Figure 19. This data shows that coated lenses (Lens Type 41) have a similar release profile as the uncoated lenses (Lens Type 47) as shown in Figure 16.

### Example 30

### Biological Efficacy of Lens Type 41

Lens Type 41 was evaluated by the method of Example 12 The amount of silver in the lenses was determined using the method of Example 15. This data is displayed in Table 6, where the control for the log reduction is a lens that is comparable to Lens Type 41, but does not contain silver.

**Table 7**

| [Ag] in ppm | 353 ± 21.7 | 358 ± 26.6 | 360 ± 21.9 | 353 ± 45.8 | 386 ± 25.3 |
|---|---|---|---|---|---|
| Log reduction | 2.58 | 2.67 | 2.52 | 2.40 | 2.42 |

### Example 31

### Comparison of the Release Profiles Lens Types 9, 14, 47, and 45

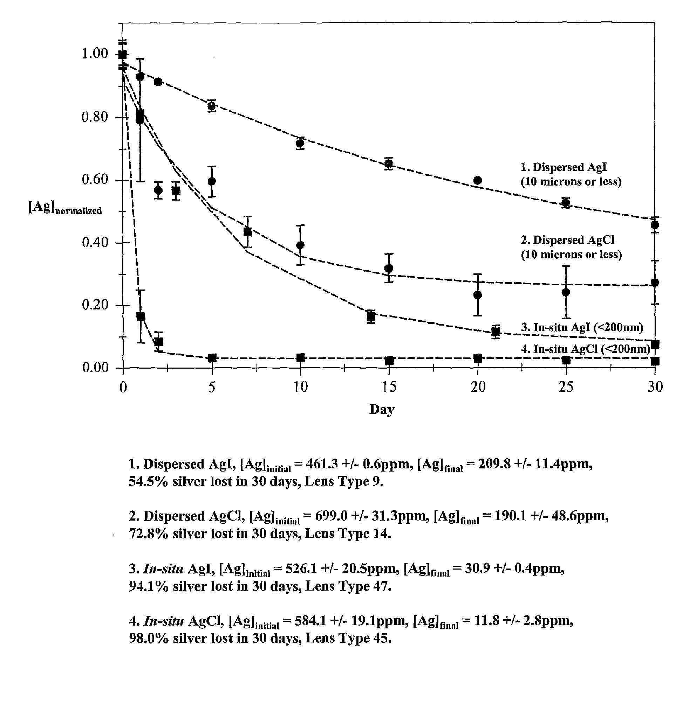

Lens Types 9, 14, 47, and 45 were evaluated to determine the release profile of the lenses. The data is presented in Figure 20 and shows that the lenses containing silver halides produced by the all methods retain silver after a period of thirty days.

### Example 32

### Preparation of Antimicrobial Lenses From Cured Lenses

Cured and hydrated lenses of Lens Type B were placed in deionized water in a blister pack. The excess deionized water was removed and sodium iodide solution (900 µL of 390 µg/mL Nal in deionized water) was added to the blister containing the lens. After 2 minutes, this solution was removed and a solution of silver nitrate was added (900 µL of 150 µg/mL silver nitrate in deionized water). After two additional minutes, the silver nitrate solution was removed and deionized water (900 µL) was added to the blister, left for approximately five minutes, and finally removed. The deionized water treatment was repeated and the lenses were transferred to glass vials containing SPS. The vials were sealed and autoclaved at 122 °C for 30 minutes and analyzed for silver content using the method of Example 1. The average silver content per lens was determined to be 178.6 ±5.4 ppm.

### Example 33

### Preparation of, Release of Metal From, and Initial Efficacy Determination of Lens Types 53-57

A hydrogel blend was made from the following monomer mix (all amounts were calculated as weight percent of the total weight of the components): 17.98% Macromer 2, 28.0% mPDMS, 14.0% TRIS, 26.0% DMA, 5.0% HEMA, 1.0% TEGDMA, 5.0% PVP, 2.0% Norbloc, 1.0% CGI 1850, and 0.02% Blue HEMA, 80 weight percent of the preceding component mixture was further diluted with diluent, 20 weight percent of, 30:70 dipropylene glycol:DPMA. Twenty grams (20g) of this blend was added to a quantity of metal salt (35.5mg manganese(II) sulfide, 25.2mg zinc(II) oxide, 30.7mg zinc(II) sulfide, 30.0mg copper(II) sulfide, and 46.0mg copper(II) phosphate, all from Aldrich) such that the final metal concentration of the hydrogel blend was approximately 1000ppm. The mixture was then sonicated in a water bath at 50°C for a minimum of 60 minutes, degassed under vacuum for 30 minutes, and then rolled on a jar roller set to about 50rpms at room temperature overnight. The mixtures were loaded into an eight-cavity lens mold of the type described in U.S. patent 4,640,489. Polymerization occurred under a nitrogen purge and was photoinitiated with visible light generated by four parallel Philips TL03 fluorescent bulbs (4 inches above the molds), at a temperature of 50°C over 30 minutes. After curing, the molds were opened and, unless noted otherwise, a minimum of three lenses from each set was manually removed from the frames and dried in individual plastic vials in a vacuum oven for three to four hours at 80°C, at a maximum pressure of five inHg, and then submitted to an independent laboratory for acid digestion and non-hydrated metal content measurements by inductively coupled plasma optical emission spectroscopy (ICP-OES).

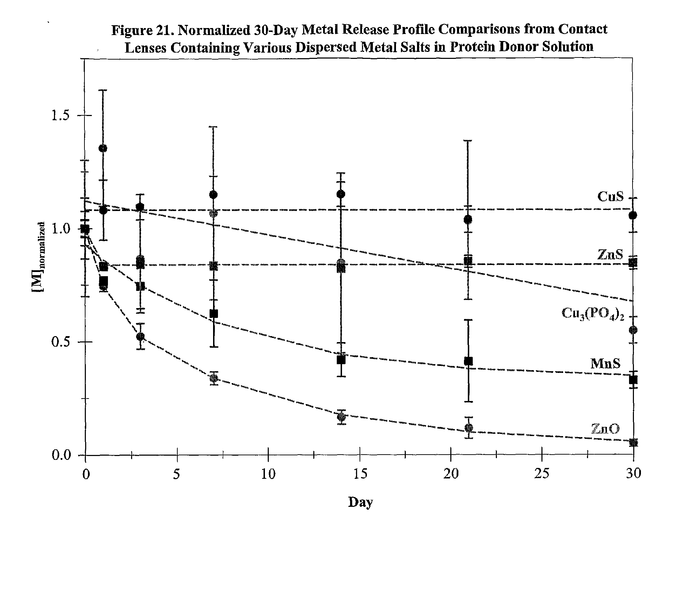

In order to obtain release profiles, lenses were manually removed from the frames and placed separately into individual plastic vials with 2.2 mL standard PD-A which was exchanged every 24 hours. The vials were kept in a tray on a plate shaker throughout the experiment, at room temperature. Triplicate samples were pulled on different days throughout the thirty-day test period, dried as described previously, and submitted to an independent laboratory for acid digestion and remaining metal content analysis by ICP-OES. Plots of the normalized release results are shown in Figure 21.

In order to prepare them for initial efficacy testing, additional lenses were released, leached, and hydrated in the following manner. The front and base curves of the plastic molds containing the cured lenses were separated manually and whichever curve the lens stuck to was placed in a 400mL glass jar. Each set of lenses, up to 20 frames or 160 lenses, was placed in their own jar, which was then filled with a 50:50 mixture of deionized H₂O:IPA (2-propanol) and left to sit overnight. The next day, the plastic curves were removed from the jar leaving behind only the lenses, the solution was replaced with 100% IPA, and the jar placed on a roller set to approximately 30 rpms and left to roll for a minimum of 30 minutes. The solution was subsequently replaced with a 50:50 mixture of deionized H₂O:IPA and then with 100% deionized H₂O, which was changed out a total of three times. Between each solution change, the jar was placed on the roller for a minimum of 30 minutes. The initial efficacy was determined by the method of Example 12 and presented in Table 8.

**Table 8**

| **Metal Salt** | **K_{SD} in pure H₂O at 25°C*** | **Molar Solubility (moles/L)** | **Non-hydrated Metal Content (ppm)** | **Hydrated Metal Content (ppm)** | **Log Reduction (Efficacy)**** |
|---|---|---|---|---|---|
| Zinc oxide | 3.87x10⁻¹⁰ | 1.97x10⁻⁵ | 1463.7(50.3) | 523.7(111.5) | 0.59 |
| Manganese sulfide | 3.00x10⁻¹⁴ | 1.73x10⁻⁷ | 1898.2(74.0) n=2 | 735.6 n=1 | 0.79 |
| Copper phosphate | 1.39x10⁻³⁷ | 3.62x10⁻⁸ | 1290.7(387.8) | 168.8(72.9) | 0.65 |
| Zinc sulfide | 2.00x10⁻²⁵ | 4.47x10⁻¹³ | 429.9(57.7) | 560.4(38.5) | 1.24 |
| Copper sulfide | 6.00x10⁻³⁷ | 7.75x10⁻¹⁹ | 2576.0(194.9) | 2993.1(145.5) | 0.79 |

| | | | | | |
|---|---|---|---|---|---|
| *CRC Handbook of Chemistry and Physics, 74th edition **At 10⁶ cfu/mL inoculum vs.Lens Type A | | | | | |

### Aspects of the invention

1. An antimicrobial ophthalmic lens comprising a metal salt and having a percent haze of less than about 200%.
2. The lens of aspect 1 wherein the formula of the metal salt is [M]ₐ [X]_{b} wherein X contains any negatively charged ion, a is ≥ 1, b is ≥ 1 and M is any positively charged metal.
3. The lens of aspect 2 wherein M is selected from the group consisting of Al⁺³, Co⁺², Co⁺³, Ca⁺², Mg⁺², Ni⁺², Ti⁺², Ti⁺³, Ti⁺⁴, V⁺², V⁺³, V⁺⁵, Sr⁺², Fe⁺², Fe⁺³, Au⁺², Au⁺³, Au⁺¹, Ag⁺², Ag⁺¹, Pd⁺², Pd⁺⁴, Pt⁺², Pt⁺⁴, Cu⁺¹, Cu⁺², Mn⁺², Mn⁺³, Mn⁺⁴, and Zn⁺².
4. The lens of aspect 2 wherein M is selected from the group consisting of Mg⁺², Zn⁺² Cu⁺¹, Cu⁺², Au⁺², Au⁺³, Au⁺¹, Pd⁺², Pd⁺⁴, Pt⁺², Pt⁺⁴ Ag⁺², and Ag⁺¹.
5. The lens of aspect 2 wherein M is selected from the group consisting of Au⁺², Au⁺³, Au⁺¹, Ag⁺², and Ag⁺¹
6. The lens of aspect 2 wherein M is selected from the group consisting of Ag⁺¹.
7. The lens of aspect 2 wherein X is selected from the group consisting of CO₃⁻² NO₃⁻¹, PO₄⁻³, Cl⁻¹, I⁻¹, Br⁻¹, S⁻² and O⁻²_{.}
8. The lens of aspect 2 wherein X is selected from the group consisting of CO₃⁻² NO₃⁻¹, Cl⁻¹, I⁻¹, and Br⁻¹.
9. The lens of aspect 2 wherein M is silver and X is selected from the group consisting of CO₃⁻² NO₃⁻¹, Cl⁻¹, I⁻¹, and Br⁻¹.
10. The lens of aspect 1 wherein the metal salt is selected from the group consisting of silver nitrate, silver sulfate, silver iodate, silver carbonate, silver phosphate, silver sulfide, silver chloride, silver bromide, silver iodide, and silver oxide.
11. The lens of aspect 1 wherein the metal salt is selected from the group consisting of silver nitrate, silver sulfate, silver iodate, silver chloride, silver bromide, and silver iodide.
12. The lens of aspect 1 wherein the diameter of the metal salt particles is less than about ten microns.
13. The lens of aspect 1 wherein the diameter of the metal salt particles is equal to or less than about 200 nm.
14. The lens of aspect 2 wherein M is silver and the amount of silver per lens is about 0.00001 to about 10 weight percent.
15. The lens of aspect 2 wherein M is silver and the amount of silver per lens is about 0.0001 to about 1.0 weight percent.
16. The lens of aspect 2 wherein M is silver and the amount of silver per lens is about 0.001 to about 0.1 weight percent.
17. The lens of aspect 1 wherein the lens formulation comprises etafilcon A, genfilcon A, lenefilcon A, polymacon, acquafilcon A, balafilcon A, galyfilcon A, senofilcon A or lotrafilcon A.
18. The lens of aspect 17 wherein the metal salt is silver chloride, silver iodide or silver bromide and the amount of silver present per lens is about 0.001 to about 0.1 weight percent.
19. The lens of aspect 1 wherein the molar solubility of the metal ion in water at about 25 °C is greater than or equal to about 2.0 x 10⁻³⁰moles/L to about less than about 20 moles/L.
20. The lens of aspect 19 wherein the molar solubility of the metal ion is greater than or equal to about 2.0 x 10⁻¹⁷ moles/L.
21. The lens of aspect 19 wherein the molar solubility of the metal ion is greater than or equal to about 9.00 x 10⁻⁹ moles/L to less than or equal to 1.0 x 10⁻⁵ moles/L when measured at 25 °C.
22. An antimicrobial lens comprising a metal complex wherein the molar solubility of the metal ions in pure water at about 25 °C is greater than or equal to about 2.00 x 10⁻³⁰moles/L.
23. The lens of aspect 22 wherein the metal ion is selected from the group consisting of Al⁺³, Co⁺², Co⁺³, Ca⁺³, Mg⁺², Ni⁺², Ti⁺², Ti⁺³, Ti⁺⁴, V⁺², V⁺³, V⁺⁵, Sr⁺², Fe⁺², Fe⁺³, Au⁺², Au⁺³, Au⁺¹, Ag⁺², Ag⁺¹, Pd⁺², Pd⁺⁴, Pt⁺², Pt⁺⁴, Cu⁺¹, Cu⁺², Mn⁺², Mn⁺³, Mn⁺⁴ and Zn⁺².
24. The lens of aspect 22 wherein the molar solubility of the metal ion is greater than or equal to about 2.0 x 10⁻¹⁷ moles/L.
25. The lens of aspect 23 wherein the molar solubility of the metal ion is greater than or equal to about 9.00 x 10⁻⁹ moles/L to less than or equal to 1.0 x 10⁻⁵ moles/L when measured at 25 °C.
26. A method of reducing the adverse events associated with microbial colonization on a lens placed in the ocular regions of a mammal comprising, placing an antimicrobial lens comprising a metal salt on the eye of a mammal.
27. The method of aspect 26 wherein the adverse events are contact ocular inflammation, contact lens related peripheral ulcers, contact lens associated red eye, infiltrative keratitis, or microbial keratitis.
28. A method of producing an antimicrobial lens comprising a metal salt wherein the method comprises mixing the metal salt with lens components to make a lens formulation and forming the lens from said lens formulation.
29. A method of preparing an antimicrobial lens comprising a metal salt, wherein the method comprises, the steps of
   (a) mixing a salt precursor with a lens formulation;
   (b) forming the lens with the product of step (a); and
   (c) treating the lens with a metal agent.
30. The method of aspect 29 wherein the salt precursor is soluble in a lens formulation at about 1 µg/mL or greater.
31. The method of aspect 29 wherein the salt precursor is selected from the group consisting of tetra-alkyl ammonium lactate, tetra-alkyl ammonium sulfate, quaternary ammonium halides, sodium chloride, sodium tetrachloro argentate, sodium iodide, sodium bromide, lithium chloride, lithium sulfide, sodium sulfide, and potassium sulfide.
32. The method of aspect 29 wherein the salt precursor is sodium iodide.
33. A method of preparing an antimicrobial lens comprising a metal salt, wherein the method comprises the steps of
   (a) mixing a metal precursor with an lens formulation ;
   (b) forming the lens ; and
   (c) treating the lens with an anion precursor.
34. The method of aspect 33 wherein the metal precursor is silver triflate, silver nitrate, copper nitrate, copper sulfate, magnesium sulfate, or zinc sulfate.
35. The method of aspect 34 wherein the anion precursor is sodium bromide, sodium chloride, or sodium iodide.
36. A method of preparing an antimicrobial lens comprising a metal salt, wherein the method comprises the steps of
   (a) treating a cured lens with a salt precursor;
   (b) treating the lens of step (a) with a metal agent under conditions to produce an antimicrobial lens having less than about 200% haze.
37. A method of preparing an antimicrobial lens comprising a metal salt, wherein the method comprises the steps of
   (b) treating cured lens of with a metal agent.
   (b) treating the lens of step (a) with a salt precursor.
38. The lens of aspect 1 having a haze value of less than 100% vs a standard CSI lens.
39. A method of preparing an antimicrobial lens comprising a metal salt, wherein the method comprises the steps of
   (a) mixing a metal with a lens formulation;
   (b) forming the lens;
   (c) treating the lens of step (b) with an oxidizing agent; and
   (d) treating the lens of step (c) with an anion precursor.
40. The lens of aspect 18 wherein the lens formulation is selected from the group consisting of acquafilcon A, galyfilcon A, senofilcon A and the metal salt is silver iodide.
41. The lens of aspect 18 wherein the lens formulation is Lens B.
42. The method of aspect 36, wherein the salt precursor is sodium iodide and the metal agent is silver nitrate.
43. The lens of aspect 1 having less than 150% haze.
44. The method of aspect 29, 33, 36, 37 or 39, wherein said lens has less than 150% haze.

## Claims

1. An antimicrobial ophthalmic lens comprising a metal salt and having a percent haze of less than 200%, wherein the formula of the metal salt is [M]ₐ [X]_{b} wherein X contains any negatively charged ion, a is ≥ 1, b is ≥ 1 and M is selected from the group consisting of Cu⁺², Cu⁺¹, Zn⁺², Mn⁺², Mn⁺³, Mn⁺⁴, Al⁺³, Co⁺², Co⁺³, Ca⁺², Mg⁺², Ni⁺², Ti⁺², Ti⁺³, Ti⁺⁴, V⁺², V⁺³, V⁺⁵, Sr⁺², Fe⁺², Fe⁺³, Au⁺², Au⁺³, Au⁺¹, Pd⁺², Pd⁺⁴, Pt⁺², and Pt⁺⁴.

2. The lens of claim 1 wherein M is selected from
the group consisting of Cu⁺², Cu⁺¹, Zn⁺², Mg⁺², Au⁺², Au⁺³, Au⁺¹, Pd⁺², Pd⁺⁴, pt⁺² and Pt⁺⁴; or
the group consisting of Au⁺², Au⁺³ and Au⁺¹.

3. The lens of claim 1 wherein X is selected from
the group consisting of S⁻², CO₃⁻² NO₃⁻¹, PO₄⁻³, Cl⁻¹, I⁻¹, Br⁻¹, and O⁻²; or
the group consisting of CO₃⁻² NO₃⁻¹, Cl⁻¹, I⁻¹, and Br⁻¹.

4. The lens of claim 1 wherein the diameter of the metal salt particles is less than ten microns, or equal to or less than 200 nm.

5. The lens of claim 1 wherein the lens formulation comprises etafilcon A, genfilcon A, lenefilcon A, polymacon, acquafilcon A, balafilcon A, galyfilcon A, senofilcon A or lotrafilcon A.

6. The lens of claim 1 wherein the molar solubility of the metal ion in water at about 25°C is greater than or equal to 2.0 x 10⁻³⁰moles/L to less than 20 moles/L, or greater than or equal to 2.0 x 10⁻¹⁷ moles/L.

7. The lens of claim 6 wherein the molar solubility of the metal ion is greater than or equal to 9.00 x 10⁻⁹ moles/L to less than or equal to 1.0 x 10⁻⁵ moles/L when measured at 25 °C.

8. A metal salt for use in a method of reducing the adverse events associated with microbial colonization on a lens placed in the ocular regions of a mammal, such as contact ocular inflammation, contact lens related peripheral ulcers, contact lens associated red eye, infiltrative keratitis, or microbial keratitis, comprising placing an antimicrobial lens comprising the metal salt on the eye of a mammal, wherein the formula of the metal salt is [M]ₐ [X]_{b} wherein X contains any negatively charged ion, a is ≥ 1, b is ≥ 1 and M is selected from the group consisting of Cu⁺², Cu⁺¹, Zn⁺², Mn⁺², Mn⁺³, Mn⁺⁴, Al⁺³, Co⁺², Co⁺³, Ca⁺², Mg⁺², Ni⁺², Ti⁺², Ti⁺³, Ti⁺⁴, V⁺², V⁺³, V⁺⁵, Sr⁺², Fe⁺², Fe⁺³, Au⁺², Au⁺³, Au⁺¹, Pd⁺², Pd⁺⁴, Pt⁺², and Pt⁺⁴.

9. A method of producing an antimicrobial lens according to claim 1 wherein the method comprises mixing the metal salt with lens components to make a lens formulation and forming the lens from said lens formulation.

10. A method of preparing an antimicrobial lens according to claim 1 wherein the method comprises, the steps of
(a) mixing a salt precursor with a lens formulation;
(b) forming the lens with the product of step (a); and
(c) treating the lens with a metal agent.

11. A method of preparing an antimicrobial lens according to claim 1 wherein the method comprises the steps of
(a) mixing a metal precursor with a lens formulation;
(b) forming the lens; and
(c) treating the lens with an anion precursor.

12. A method of preparing an antimicrobial lens according to claim 1 wherein the method comprises the steps of
(a) treating a cured lens with a salt precursor;
(b) treating the lens of step (a) with a metal agent under conditions to produce an antimicrobial lens having less than 200% haze.

13. A method of preparing an antimicrobial lens according to claim 1 wherein the method comprises the steps of
(a) treating a cured lens with a metal agent; and
(b) treating the lens of step (a) with a salt precursor.

14. A method of preparing an antimicrobial lens according to claim 1 wherein the method comprises the steps of
(a) mixing a metal with a lens formulation;
(b) forming the lens;
(c) treating the lens of step (b) with an oxidizing agent; and
(d) treating the lens of step (c) with an anion precursor.

15. The lens of claim 1 having less than 150% haze, or a haze value of less than 100% vs a standard CSI lens.
